(19) Europäisches Patentamt / European Patent Office / Office européen des brevets

(11) **EP 3 863 697 B1**

(12) **EUROPEAN PATENT SPECIFICATION**

(45) Date of publication and mention
of the grant of the patent:
**20.08.2025 Bulletin 2025/34**

(21) Application number: **19794363.2**

(22) Date of filing: **08.10.2019**

(51) International Patent Classification (IPC):
*A61M 5/14* *(2006.01)*   *A61M 5/145* *(2006.01)*
*A61M 5/168* *(2006.01)*   *A61M 5/158* *(2006.01)*

(52) Cooperative Patent Classification (CPC):
**A61M 5/16827; A61M 5/1408; A61M 5/14526;**
**A61M 5/1454; A61M 5/158;** A61M 5/14248;
A61M 2005/1405; A61M 2205/3569;
A61M 2205/3592

(86) International application number:
**PCT/US2019/055127**

(87) International publication number:
**WO 2020/076777 (16.04.2020 Gazette 2020/16)**

(54) **DRUG-DELIVERY SYSTEMS INCLUDING DRUG-DELIVERY DEVICE ASSEMBLIES FOR ATTACHING TO INFUSION NEEDLE HUBS**

MEDIKAMENTENVERABREICHUNGSSYSTEME BEINHALTEND MEDIKAMENTENVERABREICHUNGSGERÄTE ZUM AUFSETZEN AUF INFUSIONSNADELANSÄTZE

SYSTÈMES D'ADMINISTRATION DE MÉDICAMENT COMPRENANT DES ENSEMBLES DE DISPOSITIF D'ADMINISTRATION DE MÉDICAMENT DESTINÉS À ÊTRE FIXÉS À DES RACCORDS D'AIGUILLE DE PERFUSION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.10.2018 US 201862743706 P**
**24.06.2019 US 201962865340 P**

(43) Date of publication of application:
**18.08.2021 Bulletin 2021/33**

(60) Divisional application:
**25171932.4 / 4 566 644**

(73) Proprietor: **Eli Lilly and Company**
**Indianapolis, IN 46285 (US)**

(72) Inventors:
• **HARTMANN, Daniel Morris**
**Indianapolis, Indiana 46206-6288 (US)**
• **MCKEOWN, Gavin Miles**
**Indianapolis, Indiana 46206-6288 (US)**

(74) Representative: **J A Kemp LLP**
**80 Turnmill Street**
**London EC1M 5QU (GB)**

(56) References cited:
EP-A1- 2 437 817          WO-A1-2012/072555
US-A1- 2002 065 484       US-A1- 2011 160 696
US-A1- 2012 203 198       US-A1- 2017 189 621
US-B1- 6 620 138          US-B2- 8 303 545

EP 3 863 697 B1

**Description**

FIELD OF THE DISCLOSURE

The present disclosure relates to drug-delivery systems, and in particular to drug-delivery systems including drug-delivery device assemblies that are attachable to an infusion hub with a pre-inserted cannula. The drug-delivery device assemblies disclosed herein can deliver doses of a secondary drug through the pre-inserted cannula of the infusion hub.

BACKGROUND

Individuals with chronic medical conditions often use infusion sets to deliver medication from a pump to an insertion site on the body. For example, patients with insulin-dependent diabetes (also referred to simply as "diabetes") who use continuous insulin infusion therapy often use insulin infusion sets to deliver insulin through an insertion site on the abdomen, thigh, arm, lower back, or buttock. The infusion set carries insulin from the pump through a tube to a needle or cannula mounted on an insertion hub and inserted into a subcutaneous space on the patient's body.

Patients using an infusion set to deliver a medication to the insertion site may desire delivery of a second medication in addition to the primary medication. For example, patients with insulin-dependent diabetes may experience hypoglycemic events in which their glucose levels drop too low. This can happen, for instance, when the patient administers too much insulin, when the patient exercises, when the patient has not eaten for an extended time, or when the patient is asleep for an extended time. If the hypoglycemic event is mild, it may be treated by ingesting fast-acting carbohydrates. However, in cases of severe hypoglycemia and/or when the patient is unable to eat or drink, it may be treated by administering one or more therapeutic agents operative to raise a patient's glucose levels (e.g., blood glucose levels or interstitial glucose levels), such as glucagon, glucagon analogs, glucagon derivatives (simply referred to herein as "glucagon").

Glucagon may be administered using an emergency glucagon kit. Such emergency kits may comprise an injection device that stores a single, full dose of glucagon and, when pressed against a portion of the patient's body and activated, injects the entire dose via a subcutaneous needle injection. Alternatively, such emergency kits may comprise one or more vials of glucagon and a syringe that the patient may manually fill and inject. However, these emergency glucagon kits may suffer from several disadvantages.

For example, patients with diabetes are generally already burdened by the need to carry and manage various devices and supplies, such as a blood-glucose monitor (BGM), a continuous-glucose monitor (CGM), insulin pump lancing device, spare lancets, infusion sets, pump cartridges, alcohol swabs, adhesives, syringes, insulin, and/or fast-acting carbohydrates. The cognitive and emotional load of carrying a separate emergency glucagon kit in addition to all these devices and supplies may take a toll, and many patients may refuse or forget to carry an emergency glucagon kit with them.

Furthermore, emergency glucagon kits require a separate subcutaneous injection to administer glucagon (i.e., separate from a pre-inserted infusion hub delivering the primary drug), thereby requiring another injection site on the patient and increasing the risk of additional pain, infection, or other complications.

Therefore, a need exists for a delivery device assembly that is attachable to an infusion hub and is configured to deliver a secondary drug through a pre-inserted cannula.

US 2012/203198 A1 discloses a basal hub for attaching to an infusion base. The hub includes a fluid reservoir and pressure actuating device. A septum portion of the reservoir is configured to be opened by a flow cannula that is in fluid communication with an infusion cannula of the infusion base, when the basal hub is attached to the infusion base. The pressure actuating device applies pressure to the fluid reservoir, such that when the septum portion of the fluid reservoir is pierced by the by the flow cannula, liquid stored in the fluid reservoir is released from the fluid reservoir into the infusion cannula of the infusion base via the flow cannula. The basal hub is configured to deliver a basal dose of insulin during periods of inactivity, such as during sleep time.

WO2012072555A1 discloses a device to co-deliver at least two medicaments where a first reservoir containing a primary medicament is connected to a manifold and cannula and where one or more additional reservoirs are placed in fluid communication with the manifold in parallel or inline flow. The additional reservoir (s) can be located within the device or as part of an attachable medicated module that connects to an external port located on the device housing. Both medicaments are delivered through a cannula subcutaneously. The device is configured to be user wearable through attachment directly to the skin by an adhesive.

SUMMARY

The invention is defined in claim 1. Further aspects and preferred embodiments are defined in the dependent claims. Any aspects, embodiments and examples of the present disclosure which do not fall under the scope of the appended claims do not form part of the invention and are merely provided for illustrative purposes.

The present disclosure relates to drug-delivery systems including drug-delivery device assemblies that are attachable to an infusion hub with a pre-inserted cannula. In illustrated embodiments, such devices allow patients with diabetes to administer a secondary drug as needed via the cannula of the infusion hub. In some embodiments, the device is low-profile, discrete, and easy-to-assemble onto the infusion set hub. In some embodiments, such devices are relatively non-intrusive and easy-to-carry, increasing the likelihood that the patient will carry the device and thereby facilitating a discrete way to administer a secondary drug for convenience and for emergency situations.

In the illustrated embodiments, the devices are configured to administer the secondary drug through the same cannula of the infusion hub used to deliver the primary drug. Alternatively, the infusion hub may include a second pre-inserted cannula for delivery of the secondary drug in addition to the first pre-inserted cannula for delivery of the primary drug.

In addition to glucagon, the drug-delivery device assemblies of the present disclosure may be used to deliver any suitable type of secondary drugs. For example, such drug-delivery device assemblies may be used to deliver a different type of insulin than the insulin already delivered through the infusion hub. More specifically, the infusion hub may be connected to a pump and reservoir designed to deliver basal insulin (the primary drug), and the drug-delivery device assembly of the present disclosure may deliver meal-time bolus insulin (the secondary drug). As another example, drug-delivery device assemblies of the present disclosure may be used to deliver a nonsteroidal anti-inflammatory drug (NSAID). NSAIDs are a class of drugs that reduce pain, decrease fever, reduce the likelihood of blood clots, and decrease inflammation. Doses of NSAIDs to an infusion set injection site may aid in reducing or delaying inflammation in or around the site, which in turn may increase the effectiveness or prolong the life of the injection site. NSAIDs may also reduce pain in the injection site. As another example, when an infusion set is being used to deliver a chemotherapy agent for treatment of cancer, the drug delivery device assemblies of the present disclosure may contain an antiemetic (anti-nausea) drug for delivery as a secondary drug.

In a first aspect of the present disclosure there is disclosed to a drug-delivery device assembly comprising: a manifold configured to detachably couple with an infusion hub configured to subcutaneously deliver a primary drug into a patient's body, the manifold defining a fluid channel that connects a proximal surface of the manifold with a distal surface of the manifold; a screw cap having an internal surface that, when the screw cap is coupled to the manifold, opposes the distal surface of the manifold; and a flexible drug reservoir disposed between the distal surface of the manifold and the internal surface of the screw cap, the flexible drug reservoir configured to store a secondary drug, wherein the screw cap is configured to couple to the manifold using screw threads such that rotation of the screw cap relative to the manifold causes a distance between the internal surface of the screw cap and the distal surface of the manifold to decrease, thereby compressing the flexible drug reservoir such that the secondary drug is expelled from the reservoir, through the fluid channel in the manifold, and through the infusion hub.

In some embodiments of this first aspect the infusion hub is configured to deliver the primary drug via a lumen inserted subcutaneously into the patient's body; and compression of the flexible drug reservoir causes the secondary drug to be delivered subcutaneously into the patient's body via the same lumen.

In some embodiments of this first aspect the infusion hub comprises a hub septum; the proximal surface of the manifold comprises a hub-mate septum; and the drug-delivery device assembly further comprises a needle hub having a staked double-pointed needle with a proximal end and a distal end, wherein, when the drug-delivery device assembly is coupled with the infusion hub, the distal end of the double-pointed needle is configured to pierce the hub-mate septum and the proximal end of the double-pointed needle is configured to pierce the hub septum, thereby providing a passage for the secondary drug to flow from the drug-delivery device assembly into the infusion hub.

In some embodiments of this first aspect the drug-delivery device assembly further comprises a valve disposed between the flexible drug reservoir and the fluid channel in the manifold, the valve being configured to prevent the secondary drug from flowing into the fluid channel until the flexible drug reservoir is compressed by rotation of the screw cap relative to the manifold.

In some embodiments of this first aspect the flexible drug reservoir comprises at least one rigid plate affixed to a flexible film using a fluid-tight seal. In some embodiments, the rigid plate defines a first fluid passage configured to connect with the fluid channel in the manifold. In some embodiments, the rigid plate defines a second fluid passage for filling the flexible drug reservoir, the second fluid passage being sealed with a removable stopper when the drug reservoir is not being filled.

In some embodiments of this first aspect the manifold defines a second fluid passage for filling the flexible drug reservoir.

In some embodiments of this first aspect the flexible drug reservoir stores multiple doses of the secondary drug.

In some embodiments of this first aspect the drug-delivery device assembly further comprises a locking mechanism that prevents rotation of the screw cap relative to the manifold unless the mechanism is unlocked. The locking mechanism may comprise a spring-loaded pin.

In some embodiments of this first aspect the drug-delivery device assembly further comprises a wireless antenna and a circuit board communicably coupled to the wireless antenna, wherein the circuit board is configured to control rotation of the screw cap relative to the manifold in accordance with one or more wireless signals received via the wireless antenna. Some of these embodiments further comprises a screw-cap subassembly having a nut and a torsion spring coupled to the screw cap and the nut, the torsion spring being held in a pre-loaded state by a release mechanism controlled by the circuit board, wherein the circuit board is configured to release the release mechanism in accordance with the one or more wireless signals such that stored energy in the pre-loaded torsion spring causes the nut to rotate relative to the screw cap. The release mechanism may comprise at least one of an electrically-actuated mechanical switch, an electrically-actuated gear, and a fuse pin. In some of these embodiments, rotation of the nut relative to the screw cap causes the nut to translate proximally so as to compress the flexible drug reservoir. In some of these embodiments, rotation of the nut relative to the screw cap causes a piston in the screw-cap subassembly to translate proximally so as to compress the flexible drug reservoir.

In some embodiments of this first aspect the primary drug is at least one of an insulin and an insulin analog, and the secondary drug is at least one of glucagon and a nonsteroidal anti-inflammatory drug (NSAID).

In some of these embodiments of this first aspect the flexible drug reservoir has two or more compartments, each filled with a different drug constituent; and the two or more compartments are separated from one another by one or more frangible seals such that, when the screw cap is rotated relative to the manifold, positive pressure generated by compression of at least one of the compartments causes the one or more frangible seals to break, thereby allowing the drug constituents to mix.

In a second aspect of the present disclosure there is disclosed a method for using a drug-delivery device assembly to deliver a secondary drug to a patient's body via an infusion hub positioned on the patient's body for delivering a primary drug, the infusion hub having a hub septum, the method comprising: providing the drug-delivery device assembly, the assembly comprising a manifold defining a fluid channel for delivering the secondary drug, the fluid channel being connected to a hub-mate septum on a proximal surface of the manifold, and a needle hub separate from the manifold, the needle hub having a staked double-pointed needle with a proximal end and a distal end; pushing the needle hub against the proximal surface of the manifold such that the distal end of the double-pointed needle pierces the hub-mate septum of the manifold; pushing the manifold and the needle hub against the infusion hub such that the proximal end of the double-pointed needle pierces the hub septum of the infusion hub, thereby providing a passage for the secondary drug to flow from the fluid channel into the infusion hub. This method does not form part of the invention and is merely disclosed for illustrative purposes.

In some embodiments of this second aspect, the drug-delivery device assembly further comprises a screw cap with an interior surface coupled to the manifold using screw threads, and a flexible drug reservoir disposed between the interior surface of the screw cap and the manifold; wherein the method further comprises rotating the screw cap relative to the manifold to cause a distance between the internal surface of the screw cap and the manifold to decrease, thereby compressing the flexible drug reservoir such that the secondary drug is expelled from the reservoir, through the fluid channel in the manifold and the double-pointed needle in the needle hub, and through the infusion hub.

In a third aspect of the present disclosure there is disclosed a drug-delivery device assembly including an infusion hub that is configured to receive a primary drug from a primary drug-delivery device and deliver the primary drug into a patient's body. A manifold is configured to detachably couple with the infusion hub, and the manifold includes a drug channel. The drug-delivery device assembly further includes a drug reservoir that is configured to store a secondary drug, and the drug reservoir is disposed within the manifold and coupled to the drug channel. A drug plunger is movable relative to the drug reservoir. A biasing member is configured to move away from a loaded state and toward an unloaded state to move the drug plunger relative to the drug reservoir and force the secondary drug from the drug reservoir, through the drug channel, through the infusion hub, and into the patient's body.

## BRIEF DESCRIPTION OF THE DRAWINGS

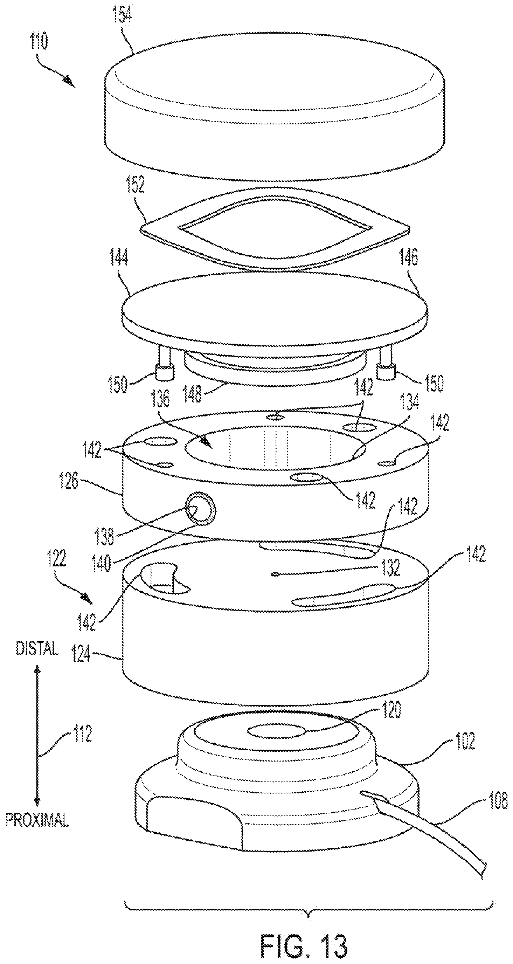

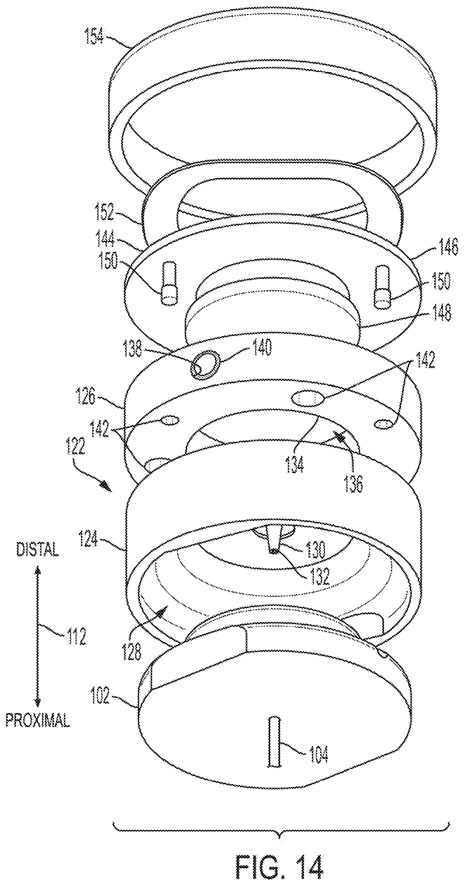

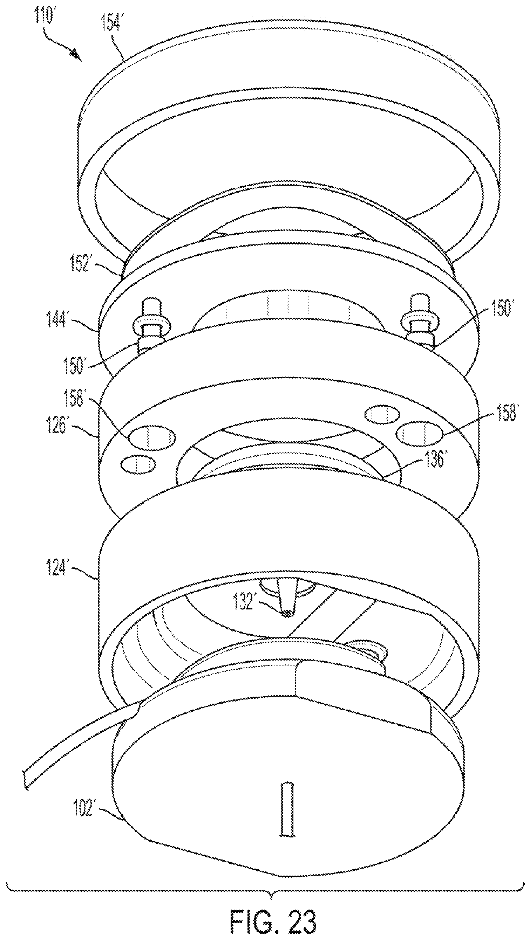

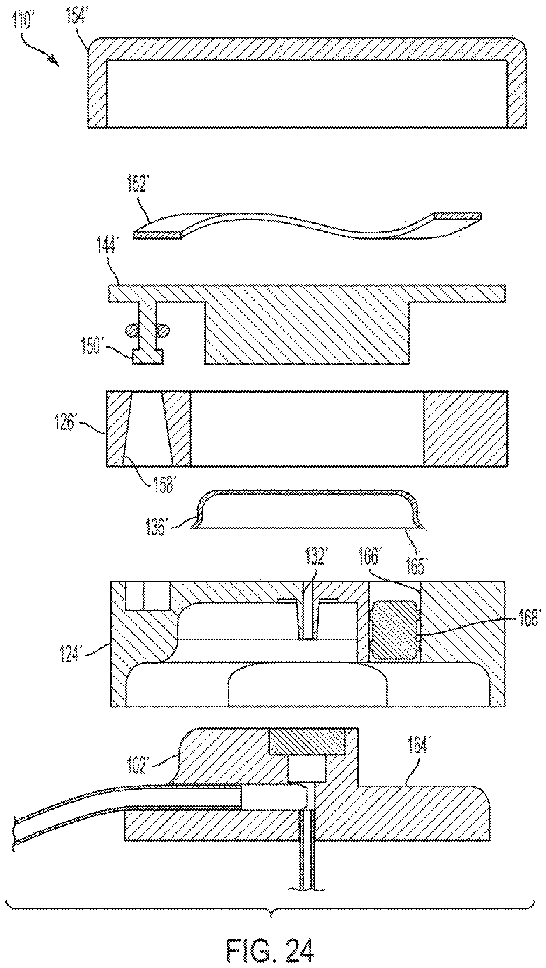

The above mentioned and other features of this present disclosure, and the manner of attaining them, will become more apparent and will be better understood by reference to the following description of embodiments of the present disclosure taken in conjunction with the accompanying drawings, wherein only FIG. 13 to FIG. 24 show drug delivery devices according to the present invention.

FIG. 1 is a perspective view of a first embodiment of a drug-delivery device assembly of the system of FIG. 12, wherein the drug-delivery device assembly is in a disassembled state.
FIG. 2 is a profile cutaway view of the drug-delivery device assembly of FIG. 1 in the disassembled state.
FIG. 3 is a profile cutaway view of the drug-delivery device assembly of FIG. 1 in an assembled state.
FIG. 4 is a perspective view of a second embodiment of a drug-delivery device assembly in a disassembled state.
FIG. 5 is a profile cutaway view of the drug-delivery device assembly of FIG. 4 in the disassembled state.
FIG. 6 is a profile cutaway view of the drug-delivery device assembly of FIG. 4 in an assembled state.

FIGS. 7A, 7B, 7C, 7D, 7E, and 7F depict an exemplary series of user steps for attaching a drug-delivery device assembly to an infusion set hub.

FIG. 8 is a perspective view of a modified screw cap sub-assembly in a disassembled state.

FIG. 9 is a profile cutaway view of the modified screw cap sub-assembly of FIG. 8, as well as other components of a drug-delivery device assembly.

FIG. 10 is a profile cutaway view of a drug-delivery device assembly that uses the modified screw cap sub-assembly, wherein the drug-delivery device assembly is in a fully assembled state.

FIG. 11 is a cutaway view of another modified screw cap sub-assembly.

FIG. 12 is a side view of an exemplary embodiment of a drug-delivery system according to the present disclosure.

FIG. 13 is an exploded top perspective view of a third embodiment of a drug-delivery device assembly and an infusion hub of the system of FIG. 12.

FIG. 14 is an exploded bottom perspective view of the drug-delivery device assembly and the infusion hub of FIG. 13.

FIG. 15 is an exploded transverse sectional view of the drug-delivery device assembly and the infusion hub of FIG. 13.

FIG. 16 is a transverse sectional view of the drug-delivery device assembly and the infusion hub of FIG. 13; a biasing member of the drug-delivery device assembly is shown in a loaded state.

FIG. 17 is a transverse sectional view of the drug-delivery device assembly and the infusion hub of FIG. 13; the biasing member of the drug-delivery device assembly is shown in an unloaded state.

FIG. 18 is a transverse sectional view of a plunger assembly exploded from a manifold of the drug-delivery device assembly of FIG. 13.

FIGS. 19-21 are transverse sectional views of the plunger assembly in different positions relative to the manifold of FIG. 18.

FIG. 22 is an exploded top perspective view of a fourth embodiment of a drug-delivery device assembly and an infusion hub according to the present disclosure.

FIG. 23 is an exploded bottom perspective view of the drug-delivery device assembly and the infusion hub of FIG. 22.

FIG. 24 is an exploded transverse sectional view of the drug-delivery device assembly and the infusion hub of FIG. 22.

FIG. 25 is a transverse sectional view of the drug-delivery device assembly and the infusion hub of FIG. 22; a biasing member of the drug-delivery device assembly is shown in a loaded state.

FIG. 26 is a transverse sectional view of the drug-delivery device assembly and the infusion hub of FIG. 22; the biasing member of the drug-delivery device assembly is shown in an unloaded state.

FIG. 27 is an exploded top perspective view of a fifth embodiment of a drug-delivery device assembly and an infusion hub according to the present disclosure.

FIG. 28 is an exploded bottom perspective view of the drug-delivery device assembly and the infusion hub of FIG. 27.

FIG. 29 is an exploded transverse sectional view of the drug-delivery device assembly and the infusion hub of FIG. 27.

FIG. 30 is a transverse sectional view of the drug-delivery device assembly and the infusion hub of FIG. 27; a biasing member of the drug-delivery device assembly is shown in a loaded state.

FIG. 31 is a transverse sectional view of the drug-delivery device assembly and the infusion hub of FIG. 27; the biasing member of the drug-delivery device assembly is shown in an unloaded state.

FIG. 32 is an exploded top perspective view of a sixth embodiment of a drug-delivery device assembly and an infusion hub according to the present disclosure.

FIG. 33 is an exploded bottom perspective view of the drug-delivery device assembly and the infusion hub of FIG. 32.

FIG. 34 is an exploded transverse sectional view of the drug-delivery device assembly and the infusion hub of FIG. 32.

FIG. 35 is a transverse sectional view of the drug-delivery device assembly and the infusion hub of FIG. 32; a biasing member of the drug-delivery device assembly is shown in a loaded state.

FIG. 36 is a transverse sectional view of the drug-delivery device assembly and the infusion hub of FIG. 32; the biasing member of the drug-delivery device assembly is shown in an unloaded state.

Corresponding reference characters indicate corresponding parts throughout the several views. Although the exemplification set out herein illustrates embodiments of the present disclosure, in several forms, the embodiments disclosed below are not intended to be exhaustive or to be construed as limiting the scope of the invention to the precise forms disclosed.

DETAILED DESCRIPTION

The present disclosure relates to drug-delivery systems including drug-delivery device assemblies, or "hub mates," that attach to an infusion hub. The infusion hub may be connected to a source of a primary drug and may be configured to deliver the primary drug subcutaneously into the patient's body via a lumen of a needle or cannula inserted into an infusion site. The drug-delivery device assemblies may carry and deliver a secondary drug into the patient's body via the needle or cannula of the infusion hub together with any of the primary drug that is also present in the infusion hub or at a time when delivery of the primary drug through the needle or cannula is halted. The primary drug or secondary drug may include one or more therapeutic agents such as insulins, insulin analogs such as insulin lispro or insulin glargine, insulin derivatives, GLP-1 receptor agonists such as dulaglutide or liraglutide, gastric inhibitory polypeptide (GIP), GIP analogs, GIP derivatives, oxyntomodulin analogs, oxyntomodulin derivatives, therapeutic antibodies such as IL-23 antibody analogs or derivatives, such as mirikizumab, IL-17 antibody analogs or derivatives, such as ixekizumab, therapeutic agents for pain related treatments, such as galcanezumab or lasmiditan, chemotherapy drugs, or any other therapeutic agent that is capable of delivery by an infusion hub. The primary drug or secondary drug may be formulated with one or more excipients.

In exemplary embodiments disclosed herein, the secondary drug includes one or more therapeutic agents such as those designed to raise a patient's glucose levels (such as glucagon), a type of insulin different than a primary insulin being delivered through the infusion hub, NSAIDs, antiemetic (anti-nausea) drugs, or any other suitable therapeutic agent that is capable of delivery through an infusion hub. Exemplary secondary drugs include, for example, meloxicam, bromfenac sodium, acetylsalicylic acid, salicylic acid, paracetamol, heparin, and dexamethasone, although other suitable drugs may be provided for delivery by the drug-delivery device assemblies of the present disclosure.

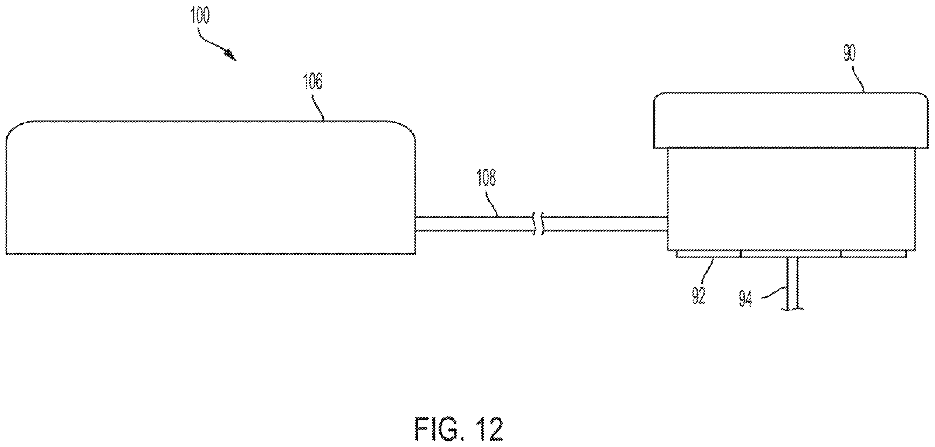

In some embodiments, the drug delivery device assembly delivers a secondary drug that is a different type of insulin than an insulin normally being delivered through the infusion set hub. For instance, the infusion set hub may be connected to a pump (FIG. 12) and reservoir designed to deliver basal insulin (the primary drug), but the patient may use the drug delivery device assembly of the present disclosure to deliver doses of rapid-acting bolus or meal-time insulin (the secondary drug).

Referring initially to FIG. 12, an exemplary embodiment of a drug-delivery system 100 is illustrated. Generally, the drug-delivery system 100 includes an infusion hub 92 that is configured to be attached, such as with an adhesive, to a patient's body at an infusion site. The infusion hub 92 includes a needle or cannula 94 that is configured to be inserted into a subcutaneous space at the infusion site. A lumen of the needle 94 is configured to deliver drugs to the patient at the infusion site. The infusion hub 92 receives a primary drug from a primary drug source 106 (for example, an insulin pump) via a primary drug conduit 108. The infusion hub 92 also receives a secondary drug from a selectively-attachable drug-delivery device assembly 90 or "hub mate." Exemplary embodiments of an infusion hub 92 and a drug-delivery device assembly 90 are illustrated and described herein.

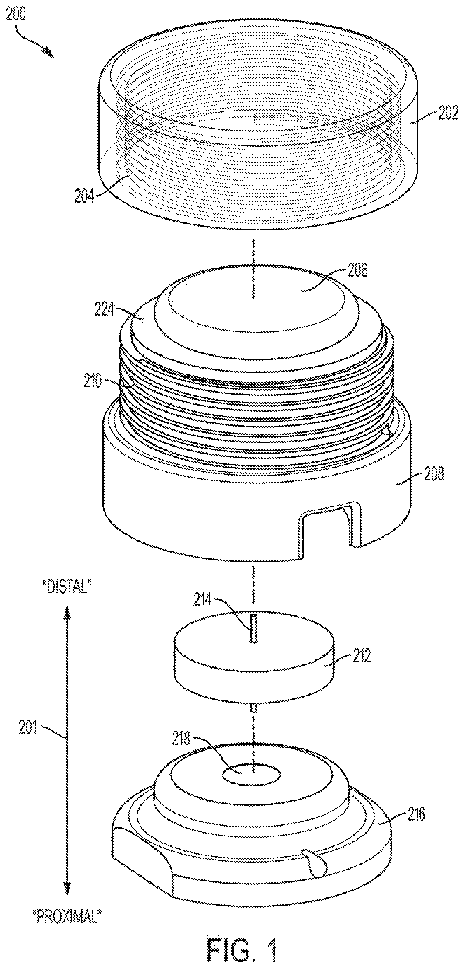

Referring now to FIG. 1, a perspective view of a first embodiment of a drug-delivery device assembly 200 of system 100 (FIG. 12) is illustrated in a disassembled state. As used in all the figures and descriptions herein, and as illustrated by double arrow 201, the terms "distal" and "proximal" refer to axial locations relative to an infusion site when a drug-delivery device assembly is oriented for use at such site, whereby, for example, proximal end of the assembly refers to the assembly end that is closest to such infusion site, and distal end of the assembly refers to the assembly end that is furthest from such infusion site. Assembly 200 includes a screw cap 202 having internal screw threads 204, a main manifold 208 having external screw threads 210, and a needle hub 212 having a double-staked needle 214. The manifold 208 of assembly 200 includes features that enable it to mate to an infusion set hub 216 configured to deliver a primary drug into a patient's body. Such features include additional screw threads, tabs that snap-fit into recesses, and/or flanges that lock into tabs. Assembly 200 further includes a flexible drug reservoir 206 disposed on a distal surface of valve capture plate 224, which is in turn disposed on a distal surface of manifold 208. Drug reservoir 206 may store, or be configured to store, a secondary drug for delivery into a patient via the infusion set hub. As discussed above, this secondary drug may include glucagon, one or more NSAIDs, an insulin or insulin derivative, or any other drug that may be stored in a flexible drug reservoir.

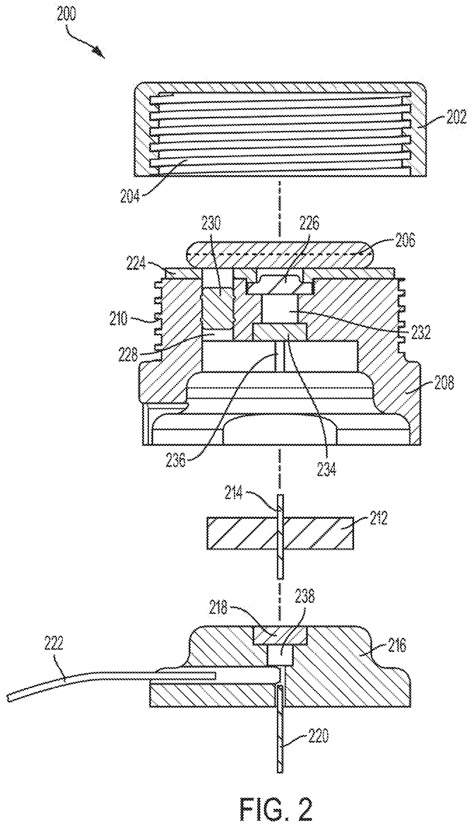

FIG. 2 provides a profile cutaway view of assembly 200 in a disassembled state. As depicted in this cutaway view, manifold 208 of assembly 200 also includes a hub-mate septum 234 that resides between fluid channels 232 and 236. Fluid channels 232 and 236 run through the axial length of manifold 208 to provide a pathway for fluids to pass from a distal surface of manifold 208 to a proximal surface of manifold 208. A valve 226 is further disposed between fluid channel 232 and the flexible drug reservoir 206, and valve 226 is held in place between valve capture plate 224 and manifold 208. Valve capture plate 224 may be joined together with manifold 208 during device assembly (e.g., by laser welding, ultrasonic welding, or adhesive bonding).

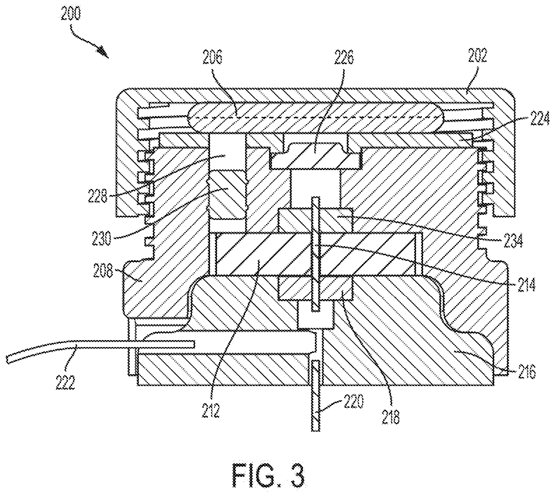

Flexible drug reservoir 206 may be made from a thin, flexible, drug-compatible film, such as cyclic olefin copolymer (COC), cyclic olefin polymers (COP), high-density polyethylene (HDPE), and/or polychlorotrifluoroethylene (PCTFE). Other materials may also be used, depending upon the drug stored therein. In some embodiments, drug reservoir 206 may comprise a single compartment that stores a single type of drug. In other embodiments, drug reservoir 206 may comprise two or more compartments, each filled with a different drug constituent. For example, reservoir 206 may comprise a first compartment stacked on top of (e.g., located distal to) a second compartment, wherein the first compartment stores a liquid diluent and the second compartment stores a lyophilized drug powder, such as glucagon powder. This arrangement is illustrated by the horizontal dashed line drawn through the center of reservoir 206 in FIGS. 2 and 3. In other embodiments (not shown), the two or more compartments may be located next to each other in the same plane, such that neither compartment is distal or proximal relative to the other compartment. When drug reservoir 206 comprises two or more compartments, the compartments may be separated from one another by one or more frangible seals.

Drug reservoir 206 may be heat-sealed or otherwise affixed in a fluid-tight manner to valve capture plate 224. In this way, valve capture plate 224 provides a rigid bottom surface on which flexible drug reservoir 206 rests. Valve capture plate 224 and manifold 208 may define a through-hole 228 which may be used to fill reservoir 206. After filling, through-hole 228 may be stopped by an elastic stopper 230.

Infusion set hub 216 is connected to a source of a primary drug (e.g., device 106 of FIG. 12) via tube 222. The source of the primary drug may comprise an infusion pump, an infusion bag, a drug reservoir, or any other component capable of supplying the primary drug. While in use, the primary drug passes through tube 222 and into infusion set hub 216, where it is delivered into the patient's body via lumen 220, which may be a needle, catheter, or cannula. Infusion set hub 216 further includes a hub septum 218 disposed on a distal surface of hub 216; hub septum 218 is also connected to lumen 220 via secondary drug channel 238.

FIG. 3 provides a profile cutaway view of assembly 200 in an assembled state. When fully assembled, screw cap 202 mates with manifold 208 such that internal screw threads 204 on screw cap 202 engage with external screw threads 210 on manifold 208. The fluid drug reservoir 206 is situated between an internal, proximal surface of screw cap 202 and a distal surface of valve capture plate 224. When assembly 200 is mated with infusion hub 216, needle hub 212 is held in place between a proximal surface of manifold 208 and a distal surface of hub 216. A distal end of double-staked needle 214 pierces hub-mate septum 234 while a proximal end of double-staked needle 214 pierces hub septum 218. In this way, double-pointed needle 214 provides a fluid conduit between the manifold 208 and the infusion hub 216.

To dispense the secondary drug from the drug reservoir 206, a user (e.g., the patient, a caregiver, and/or a healthcare professional) rotates screw cap 202 relative to manifold 208. Due to the engagement between internal screw threads 204 and external threads 210, rotation of the screw cap causes a distance between the internal surface of the screw cap 202 and the distal surface of manifold 208 to decrease. This decrease in distance compresses the flexible drug reservoir 206 such that the secondary drug is expelled from the reservoir 206, through valve 226, fluid channel 232, hub-mate septum 234, double-staked needle 214, hub septum 218, secondary drug channel 238, and lumen 220, and into the patient's body. The minimum dose that can be dispensed from assembly 200 will depend upon the thread size and pitch of the screw cap threads and the size of the area of the flexible drug reservoir 206 that is compressed when the screw cap 202 is rotated relative to manifold 208. The accuracy of the dose will depend upon the thread pitch, tolerances on the parts, and the uniformity with which the flexible reservoir 206 is compressed by the screw cap 202.

In embodiments where reservoir 206 includes a first compartment storing a liquid diluent that is stacked on top of (i.e., located distal to) a second compartment storing a lyophilized drug powder, compression of reservoir 206 generates sufficient positive pressure within the first compartment to break a frangible seal between the first and the second compartment. When the frangible seal is broken, the diluent flows from the first compartment into the second compartment to mix with the lyophilized drug powder stored in the second compartment. The mixture of the diluent with the lyophilized drug powder creates a reconstituted liquid drug mixture that is then expelled from the second compartment and into the patient's body along the fluid path described previously.

Screw cap 202 may include a lock (not shown) that may take the form of, for example, a spring-loaded pin. The lock, when engaged, prevents the cap 202 from rotating with respect to the manifold 208. In some cases, reservoir 206 may hold only enough secondary drug for a single dose-in such cases, the user may rotate cap 202 until it rotates no further with every use. After every such use, the reservoir 206 will need to be replaced or re-filled. In some cases, reservoir 206 may hold enough secondary drug for multiple doses-in such cases, the user may rotate cap 202 partially with every dose, and optionally engage the lock between doses to prevent the cap 202 from rotating further. The user may also rotate the cap 202 by different amounts to administer different amounts of secondary drug. The cap 202 and/or external surface of manifold 208 may also include markings that indicate to a user how far he or she should turn the cap 202 in order to administer a certain amount of secondary drug. For instance, the markings may indicate that a half-rotation (180°) or full-rotation (360°) of cap 202 relative to manifold 208 will result in a dose of 1 unit of secondary drug.

Valve 226 may be configured to open only when compression of the drug reservoir 206 generates sufficient positive pressure of the secondary drug. The cracking pressure of valve 226 may be tailored to be high enough that it prevents the secondary drug from being dispensed due to environmental changes, such as heating and cooling of thermoplastic parts, and/or atmospheric pressure changes. The valve also prevents backflow of liquid into reservoir 206. During storage, use, or filling, the secondary drug may come into fluid contact with various surfaces of device assembly 200, such as valve 226, stopper 230, and the inside surfaces of fluid channel 232, hub-mate septum 234, double-staked needle 214, hub septum 218, secondary drug channel 238, lumen 220, and through-hole 228. These surfaces may be made from or coated with drug-compatible materials, such as fluoroelastomer (FKM), chlorobutyl bromobutyl, silicone, or other such elastomers.

Table 1 below provides an exemplary set of parameters for assembly 200.

| Diameter of drug reservoir 206 | 22.0 | mm |
|---|---|---|
| Area of drug reservoir | 380.1 | mm$^2$ |
| Height of cylindrical drug reservoir that will hold 1 mL | 2.6 | mm |

TABLE 1: Exemplary parameters for assembly 200

| Height of reservoir needed to hold 1 mL of drug product (mm) | Thread pitch (Threads / in) | Thread pitch (Threads/mm) | Distance traveled per full revolution (mm) | Drug dispensed per full turn of screw cap (uL) | Drug dispensed per 1/4 turn (uL) |
|---|---|---|---|---|---|
| 2.6 | 56 | 2.20 | 0.45 | 174 | 44 |
| 2.6 | 40 | 1.57 | 0.64 | 244 | 61 |
| 2.6 | 32 | 1.26 | 0.79 | 305 | 76 |
| 2.6 | 28 | 1.10 | 0.91 | 349 | 87 |

Since assembly 200 utilizes the same lumen 220 for delivery of both the primary and the secondary drug, there is a volume of space within infusion set hub 216 in which the two drugs may mix. This volume of space includes the volume where secondary drug channel 238 intersects with the drug channel carrying primary drug supplied by tube 222, as well as the volume within lumen 220. In some embodiments, it may be advantageous to minimize the volume of this space by making the fluid channels small at this union. With appropriate design (e.g., with channels having less than 0.015 inches inner diameter), the total volume of space shared by the primary and the secondary drug may be kept to less than three microliters.

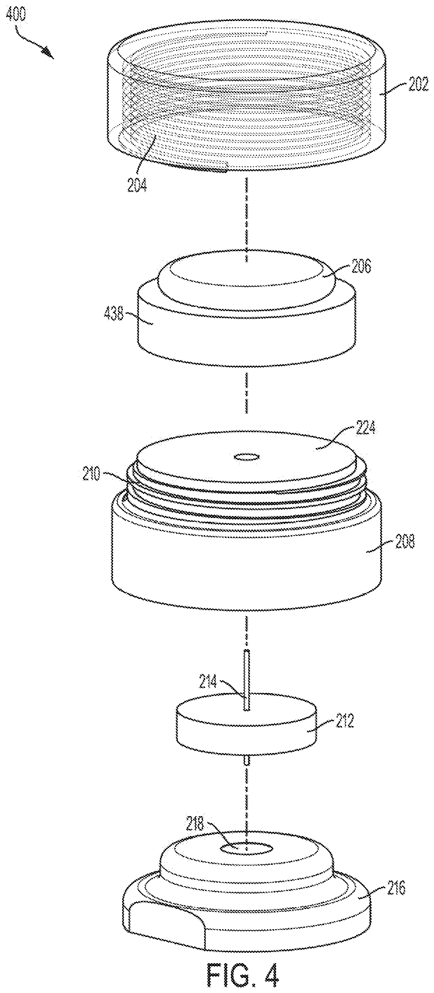

FIG. 4 provides a perspective view of a second embodiment of a drug-delivery device assembly 400 in a disassembled state. Assembly 400 is similar to assembly 200, and like components of the two assemblies have same reference numerals. In assembly 400, flexible drug reservoir 206 is not integrated with manifold 208 but instead rests on a reservoir bottom 438 that is separable from manifold 208.

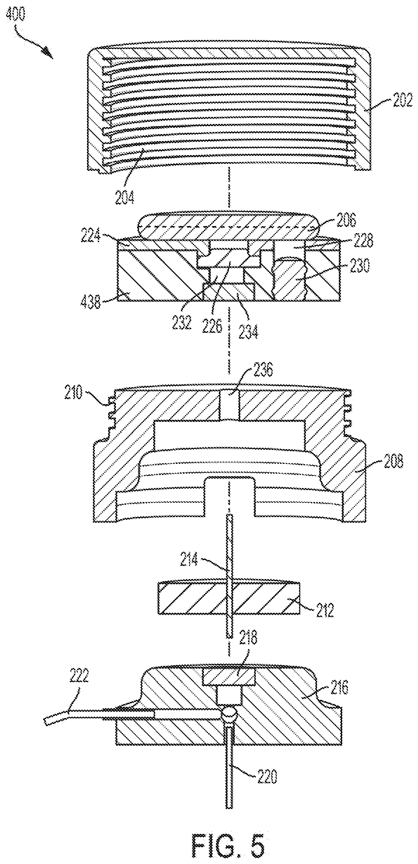

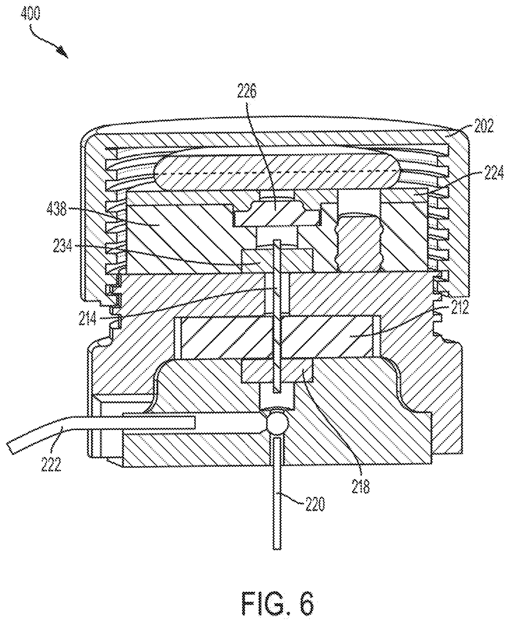

FIG. 5 provides a profile cutaway view of assembly 400 in a disassembled state, while FIG. 6 provides a profile cutaway view of assembly 400 in an assembled state. In these views, it can be seen that reservoir bottom 438 includes the valve capture plate 224, valve 226, fluid channel 232, hub-mate septum 234, through-hole 228, and stopper 230 described above. The manifold 208 includes only fluid channel 236 described above. The configuration of components in assembly 400 makes filling and storage of reservoir 206 easier. Unlike assembly 200, reservoir 206 in assembly 400 is not integrally attached to manifold 208. Since reservoir 206 and reservoir bottom 438 can be detached from manifold 208, the reservoir 206 and reservoir bottom 438 can be separately transported, stored, handled, filled, and/or re-filled. This increases the likelihood that transportation, storage, and handling of reservoir 206 is easy and convenient, particularly if the drug(s) stored in reservoir 206 must be kept at a cold temperature during manufacturing, assembly, and distribution.

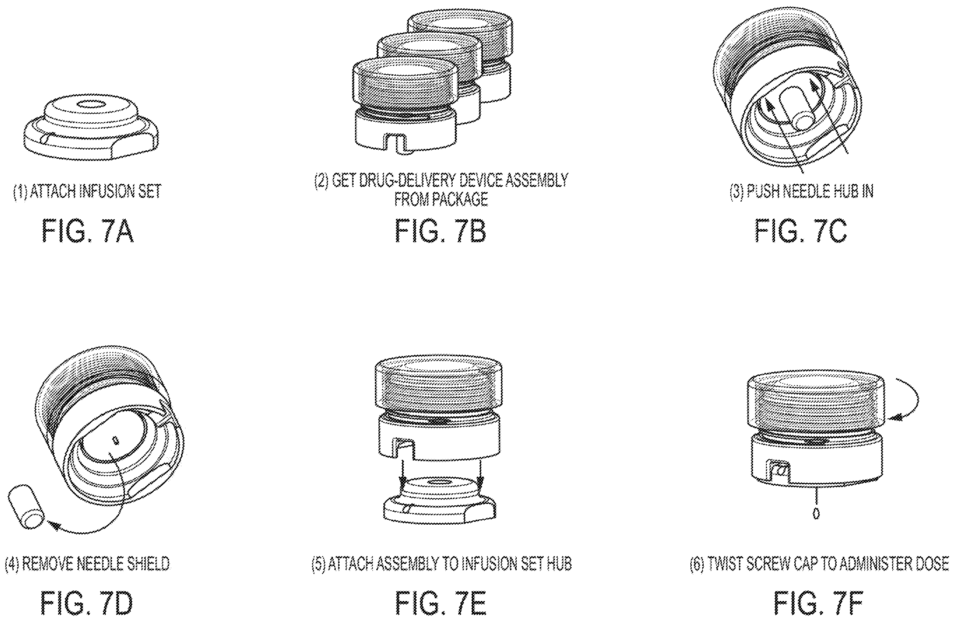

For both assembly 200 and assembly 400, to preserve the sterility of the secondary drug stored in reservoir 206, it will typically be advantageous to maintain hub-mate septum 234 in an un-pierced state during storage. However, for ease of use and/or assembly, the drug-delivery device assembly may be packaged in a way that positions the distal end of double-pointed needle 214 directly below hub-mate septum 234. An exemplary set of user steps for piercing hub-mate septum 234 and for attaching a drug-delivery device assembly to an infusion set hub is depicted in FIGS. 7A-7F. This set of user steps applies to both assemblies 200, 400, as well as other suitable embodiments of the drug-delivery device assembly.

As illustrated in FIG. 7A, a user may first attach an infusion set hub onto his or her body. As discussed above, attaching the infusion set hub may comprise inserting a needle, catheter or cannula into an infusion injection site on the user's body, such as on the user's abdomen, thigh, arm, lower back, or buttock. Although not shown in FIG. 7A, the infusion set hub may also be connected to a pump or a source of a primary drug by a tube.

As illustrated in FIG. 7B, the user may then retrieve a drug-delivery device assembly from packaging. The assembly may be packaged such that the needle hub 212 having the double-staked needle 214 is positioned directly below manifold 208, such that the distal end of needle 214 is positioned immediately below (but does not pierce) hub-mate septum 234.

As illustrated in FIG. 7C, the user may then push needle hub 212 "in", i.e., in the distal direction. This pushes the needle hub 212 against a proximal surface of manifold 208, and also causes the distal end of double-staked needle 214 to pierce hub-mate septum 234.

As illustrated in FIG. 7D, the user may then remove a needle shield (not shown in FIGS. 1-6) from the proximal end of needle 214. The needle shield may be a protective cover made of plastic or some other material that covers the proximal end of double-staked needle while the drug-delivery device assembly is in storage. Once the needle shield is removed, the proximal end of needle 214 is exposed.

As illustrated in FIG. 7E, the user may then attach the assembly to the infusion set hub by pushing the assembly down (i.e., in the proximal direction) against the distal surface of the infusion set hub. Pushing the assembly down causes the proximal end of needle 214 to pierce the hub septum 218, thereby establishing a conduit for the secondary drug to flow from the reservoir 206, into the infusion set, and from there into the patient's body.

As illustrated in FIG. 7F, once the drug-delivery device assembly has been attached to the infusion set hub, the user may then twist the screw-cap 202 to administer a dose, as described previously.

In the embodiments described so far, the drug-delivery device assembly is operated manually; that is, the user is responsible for manually actuating the assembly to deliver a dose of the secondary drug. However, in other embodiments, the assembly may also be operated automatically to, for example, dispense the secondary drug in response to a wireless signal from an external device, such as a glucose monitor or a mobile device that is receiving blood glucose values from a glucose monitor.

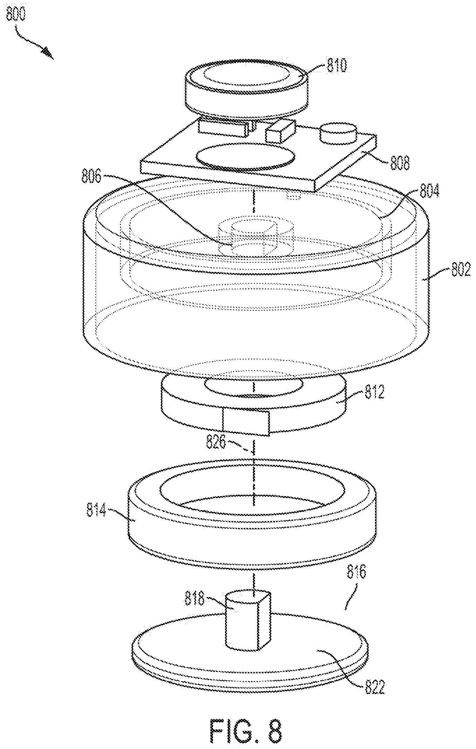

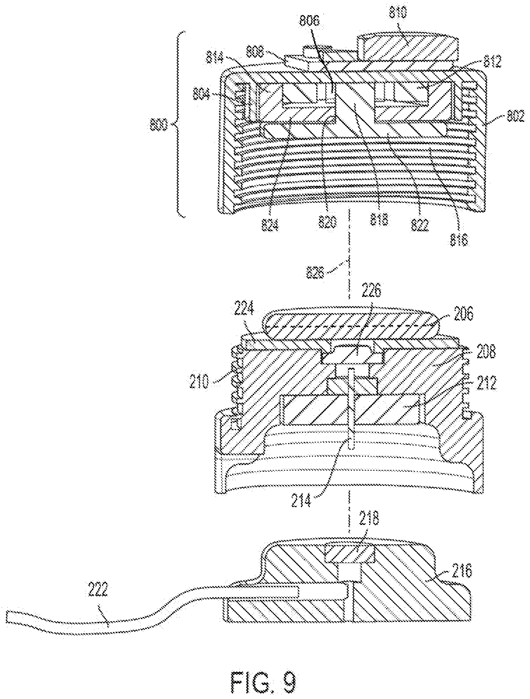

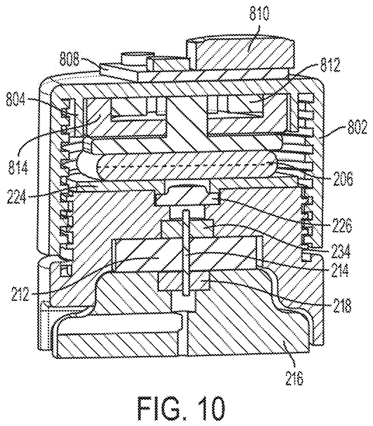

An example of one such embodiment is illustrated in FIGS. 8, 9, and 10. FIG. 8 illustrates a perspective view of a modified screw cap sub-assembly 800 in a disassembled, exploded state. FIG. 9 illustrates a profile, cutaway view of screw cap sub-assembly 800 in an assembled state, as well as other previously-described components of a drug-delivery device assembly. FIG 10 illustrates a profile cutaway view of a drug-delivery device assembly that uses screw cap sub-assembly 800, wherein the drug-delivery device assembly is in a fully assembled state.

Referring to FIG. 8, screw cap sub-assembly 800 includes a screw cap 802, on top of which is arranged a printed circuit board (PCB) 808 having a battery 810. Screw cap 802 also includes an internal pocket 804 sized to accommodate torsion spring 812, nut 814, and piston 816. As best seen in FIG. 9, nut 814 includes a proximal surface 824 that defines an opening 820. As illustrated in both FIGS. 8 and 9, piston 816 includes a piston plate 822 and a piston rod 818 perpendicular to piston plate 822. When screw cap sub-assembly 800 is assembled, piston rod 818 fits through opening 820 defined in proximal surface 824 of nut 814. At least a portion of the surface of piston rod 818 includes external screw threads that engage with internal screw threads situated on the internal surface of opening 820, such that rotation of piston rod 818 about longitudinal axis 826 relative to nut 814 causes piston rod 818 (and therefore piston 816) to translate in the proximal or distal direction along longitudinal axis 826 relative to nut 814.

When assembled, piston plate 822 may directly abut proximal surface 824 as shown in FIG. 9, or there may be a gap between proximal surface 824 and piston plate 822. Torsion spring 812 fits within nut 814 on top of proximal surface 824, and the combined piston 816, nut 814, and torsion spring 812 fit within the internal pocket 804 of screw cap 802. When assembled, the distal end of piston rod 818 fits within anti-rotation pocket 806 on the bottom (proximal) side of screw cap 802.

As best seen in FIG. 8, piston rod 818 illustratively is shaped like a rounded wedge and is sized and shaped to fit snugly within anti-rotation pocket 806. When inserted into pocket 806, the shape of the two components prevents piston 816 from rotating about longitudinal axis 826 relative to screw cap 802. The aforementioned external screw threads on piston rod 818 (that engage with the internal screw threads in opening 820) are situated on the rounded portion of the rounded wedge shape. Thus, when assembled, piston 816 may translate along longitudinal axis 826 relative to screw cap 802, but may not rotate relative to screw cap 802.

One end of torsion spring 812 (e.g., the central end) is attached to screw cap 802 (potentially via a tab or spindle that protrudes proximally from the proximal surface of screw cap 802), while the other end of torsion spring 812 (e.g., the external end) is attached to an internal surface of nut 814. When assembled, torsion spring is held in a pre-loaded, coiled state by a release mechanism controlled by PCB 808. This release mechanism (not shown) may take the form of a fuse pin that prevents torsion spring 812 from uncoiling - the PCB 808 may release the release mechanism by using stored energy from battery 810 to heat and melt the fuse pin, thus releasing torsion spring 812 and allowing it to uncoil. Alternatively, the release mechanism may take the form of a mechanical micro-switch that acts as a latch to hold the torsion spring 812 in place - when the micro-switch is moved, the torsion spring 812 is unlatched and allowed to uncoil. In yet other embodiments, the release mechanism may take the form of an actuated gear that can control the extent to which torsion spring 812 is allowed to uncoil. Other release mechanisms known in the art may also be used to hold the torsion spring 812 in its pre-loaded, coiled state until released by PCB 808. PCB 808 may trigger the release mechanism in response to an instruction to administer some or all of the secondary drug in reservoir 206. This instruction may be based on a wireless signal received from an external device (e.g., a smartphone or glucose monitor), based on the push of a button situated on drug-delivery device assembly 200, and/or based on internal logic programmed or embodied on PCB 808.

When the PCB 808 triggers the release mechanism, the uncoiling of the pre-loaded torsion spring 812 causes nut 814 to rotate about longitudinal axis 826 relative to screw cap 802. Since piston 816 is prevented from rotating relative to screw cap 802 by the interface between piston rod 818 and anti-rotation pocket 806, nut 814 also rotates relative to piston 816. Rotation of nut 814 relative to piston 816 causes piston 816 to translate in the proximal direction along longitudinal axis 826 due to the aforementioned engagement between the external screw threads on piston rod 818 and the internal screw threads in opening 820. When the piston 816 translates in the proximal direction, it compresses drug reservoir 206, thus dispensing the secondary drug along the previously-described fluid pathway into the patient. In this way, screw cap sub-assembly 800 allows the drug-delivery device assembly to automatically dispense the secondary drug (e.g., in response to a wireless instruction or signal from an external device) without requiring the user to manually actuate or twist the screw cap.

Although FIGS 8, 9, and 10 depict modified screw cap sub-assembly 800 as being used with the drug-delivery device assembly 200 disclosed herein with FIGS. 1-3, screw cap sub-assembly 800 may also be used with assembly 400 of FIGS. 4-6. For ease of illustration and explanation, some features within manifold 208 have been omitted from FIGS. 9 and 10, e.g., through-hole 228 and stopper 230. It is understood that these features may also be present in some embodiments.

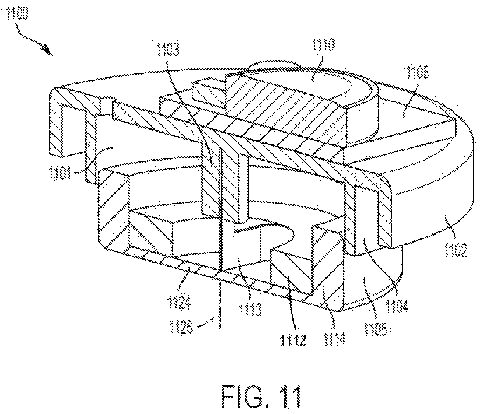

FIG. 11 illustrates yet another embodiment of a screw cap that allows for automatic dispensing of the secondary drug from a drug delivery device assembly. This modified screw cap may also be used with either of drug-delivery device assemblies 200, 400. FIG. 11 provides a cutaway view of another modified screw cap sub-assembly 1100. Assembly 1100 includes a screw cap 1102, on top of which is arranged a printed circuit board (PCB) 1108 having a battery 1110. Screw cap 1102 also includes an internal pocket 1104 sized to accommodate torsion spring 1112 and nut 1114. Unlike screw cap sub-assembly 800 depicted in FIGS. 8, 9, and 10, modified screw top assembly 1100 does not include a piston.

Torsion spring 1112 includes a tab 1113 that extends from a central end of spring 1112. Tab 1113 is attached to a central spindle portion 1103 of screw cap 1102. The other, external end of spring 1112 is attached to an interior surface of nut 1114. The external surface 1105 of nut 1114 includes external screw threads that engage with internal screw threads disposed on interior surface 1101 of pocket 1104. The engagement between these screw threads causes nut 1114 to translate in the proximal or distal direction when nut 1114 is rotated relative to screw cap 1102 about longitudinal axis 1126.

Similar to the screw top assembly 800 described herein, the torsion spring 1112 is held in a pre-loaded, coiled state by a release mechanism controlled by PCB 1108. This release mechanism may take the form of any of the release mechanisms described above in relation to screw top assembly 800. When the PCB 1108 triggers the release mechanism, the uncoiling of the pre-loaded torsion spring 1112 causes nut 1114 to rotate about longitudinal axis 1126 relative to screw cap 1102. Rotation of nut 1114 relative to screw cap 1102 causes nut 1114 to translate in the proximal direction along longitudinal axis 1126 due to the aforementioned engagement between the external screw threads on surface 1105 of nut 1114, and the internal screw threads on internal surface 1101 of pocket 1104. When the nut 1114 translates in the proximal direction, a proximal surface 1124 of nut 1114 compresses drug reservoir 206 (not shown), thus dispensing the secondary drug along the previously-described fluid pathway into the patient. In this way, screw cap sub-assembly 1100 also allows the drug-delivery device assembly to automatically dispense the secondary drug (e.g., in response to a wireless instruction or signal from an external device) without requiring the user to manually actuate or twist the screw cap.

FIGS. 13-15 are exploded views of the infusion hub 102 and the drug-delivery device assembly 90 of FIG. 12 according to another exemplary embodiment. As used in all the figures and descriptions herein, and as illustrated by double arrow 112, the terms "distal" and "proximal" refer to axial locations relative to an infusion site when a drug-delivery device assembly 110 and an infusion hub 102 are oriented for use at such site, whereby, for example, proximal end of the assembly refers to features that are closer to the infusion site, and distal end of the assembly refers to features that are farther from the infusion site.

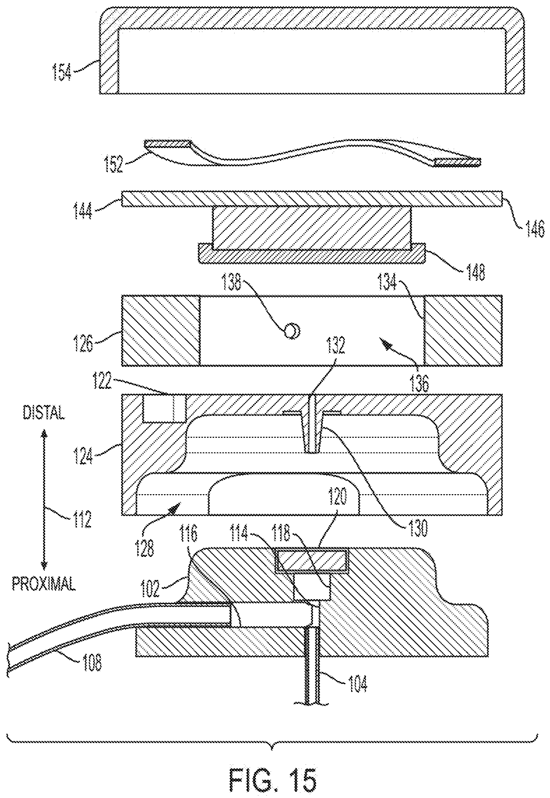

Referring to FIG. 15, the infusion hub 102 includes an internal drug-delivery channel 114 coupled to the needle 104. The drug-delivery channel 114 is also coupled to a primary drug channel 116, and the primary drug channel 116 is coupled to the primary drug conduit 108. The drug-delivery channel 114 is further coupled to a secondary drug channel 118, and the secondary drug channel 118 carries a pierceable septum 120.

Referring again to FIGS. 13-15, the drug-delivery device assembly 110 includes a manifold 122 that carries a secondary drug and is selectively attachable to the infusion hub 102. The manifold 122 may be made of any of various appropriate materials, such as cyclic olefin copolymer (COC), cyclic olefin polymers (COP), high-density polyethylene (HDPE), and/or polychlorotrifluoroethylene (PCTFE). The manifold 122 includes a first, or lower, manifold portion 124 and a second, or upper, manifold portion 126. The lower manifold portion 124 includes a lower chamber 128 that receives the infusion hub 102, and a protrusion 130 is disposed with the lower chamber 128. The protrusion 130 pierces the septum 120 of the infusion hub 102, and the protrusion 130 includes a manifold drug channel 132 for delivering the secondary drug to the secondary drug channel 118 of the infusion hub 102. The upper manifold portion 126 includes an inner wall 134 that defines a drug reservoir 136. The drug reservoir 136 initially receives the secondary drug via a fill port 138, which receives a stopper 140 after filling the drug reservoir 136. The drug reservoir 136 is coupled to the manifold drug channel 132 of the lower manifold portion 124.

In the illustrated embodiment, the manifold 122 also includes features that facilitate delivering the secondary drug continuously to a patient over a relatively long period. Generally, these features include one or more resistance channels 142 (illustratively, three resistance channels 142) that carry a motion-inhibiting or resistance medium (shown elsewhere), such as a high-viscosity liquid/gel for example. The high-viscosity liquid may be Newtonian having a constant velocity, such as glycerol for example. The high-viscosity liquid may be thixotropic wherein the viscosity decreases in shear. An example of a thixotropic liquid is a medical-grade "grease", such as a grease manufactured by Nye Lubricants (Fairhaven, MA). These optional features are described in further detail below. In other embodiments, the manifold 122 does not include such resistance channels configured to slow drug delivery.

The drug-delivery device assembly 110 further includes a plunger assembly 144 that is movably coupled to the manifold 122. Generally, the plunger assembly 144 may include any appropriate drug-compatible materials, such as FKM, chlorobutyl, bromobutyl, or silicone. The plunger assembly 144 includes a plunger base 146 and a drug plunger 148, and one or more resistance plungers 150 (illustratively, three resistance plungers 150) extend proximally from the plunger base 146. The drug plunger 148 and the resistance plungers 150 are movable together with the plunger base 146. The drug plunger 148 is movably and sealingly received in the drug reservoir 136 of the manifold 122. As such and as described in further detail below, the drug plunger 148 is proximally movable in the drug reservoir 136 to force the secondary drug from the drug reservoir 136, through the manifold drug channel 132, through the infusion hub 102, and into the patient's body together with any of the primary drug that is also present in the infusion hub 102. The resistance plungers 150 are movably and sealingly received in the resistance channels 142. As such and as described in further detail below, the resistance plungers 150 are proximally movable in the resistance channels 142 to move the resistance medium in the resistance channels 142, and the resistance medium thereby provides resistance to movement of the plunger assembly 144 relative to the manifold 122. In some embodiments, the resistance provided by the resistance medium facilitates delivering secondary drugs continuously to a patient over a relatively long time period.

With continued reference to FIGS. 13-15, the drug-delivery device assembly 110 also includes a biasing member 152 that is disposed between the plunger assembly 144 and a cover 154 coupled to the manifold 122. The biasing member 152 releases stored energy, or, stated another way, moves away from a loaded state and toward an unloaded state (as used herein, a "loaded state" and an "unloaded state" are used as relative terms to describe states in which a biasing member stores relatively high and relatively low amounts of energy, respectively), to move the plunger assembly 144 proximally relative to the manifold 122 and, as described in further detail below, facilitate delivery of the secondary drug to the patient. The biasing member 152 may take various forms. For example and as shown in FIGS. 13-15, the biasing member 152 may be a compression spring, more specifically a wave washer. In such embodiments, the wave washer is relatively compressed in the loaded state and relatively uncompressed in the unloaded state. Other types of biasing members 152 are described in further detail below and shown elsewhere.

In some embodiments, the drug-delivery device assembly 110 also includes a detachable or releasable locking mechanism (not shown) for initially holding the biasing member 152 in a loaded state. Such mechanisms may take a variety of forms. In one example, a locking mechanism includes a detachable pin that extends through the cover 154 and into the plunger assembly 144 to hold the biasing member 152 in a loaded state between the cover 154 and the plunger assembly 144. In another example, the cover 154 includes tabs that lock to corresponding tabs on the top portion of the plunger assembly 144 to hold the biasing member 152 in a loaded state. When the cover 154 is rotated with respect to the biasing member 152, the interlocking tabs are freed thereby releasing the biasing member 152.

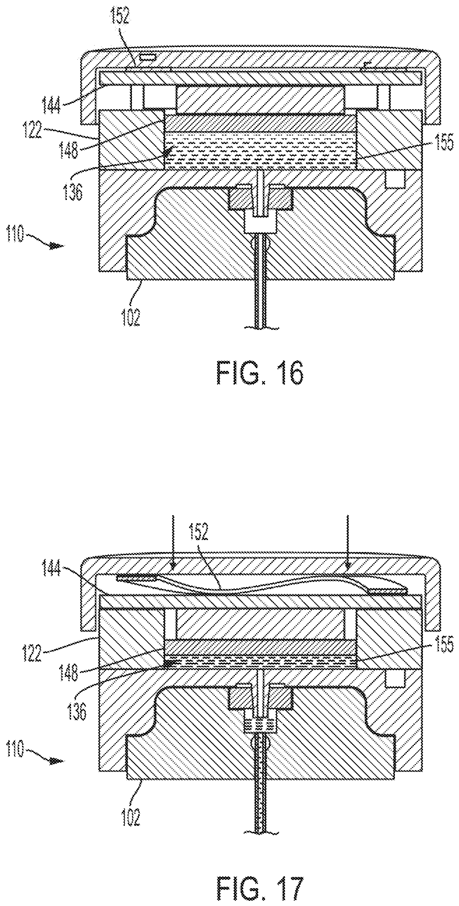

FIGS. 16 and 17 illustrate the drug-delivery device assembly 110 and the infusion hub 102 with the biasing member 152 in different states. More specifically, FIG. 16 illustrates the biasing member 152 in a loaded state, and FIG. 17 illustrates the biasing member 152 in an unloaded state. As described above, the biasing member 152 moves away from the loaded state and toward the unloaded state to move the plunger assembly 144 proximally relative to the manifold 122. As such, the biasing member 152 (a) moves the drug plunger 148 in the drug reservoir 136 to force the secondary drug 155 from the drug reservoir 136, through the manifold drug channel 132, through the infusion hub 102, and into the patient's body together with any primary drug that is also present in the infusion hub 102; and (b) moves the resistance plungers 150 in the resistance channels 142 to move the resistance medium in the resistance channels 142 (all shown elsewhere), and the resistance medium thereby provides resistance to movement of the plunger assembly 144 relative to the manifold 122.

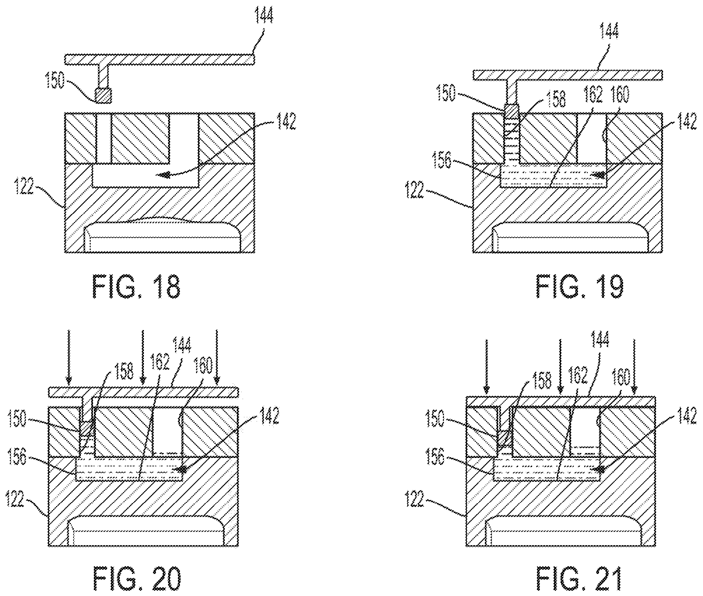

FIG. 18 illustrates the plunger assembly 144 exploded from the manifold 122 of the drug-delivery device assembly 110, and the resistance medium is shown as being absent from one of the resistance channels 142 for clarity. FIGS. 19-21 illustrate the plunger assembly 144 and one of the resistance plungers 150 in different positions relative to the manifold 122. More specifically, FIG. 19 illustrates the position of the plunger assembly 144 in a loaded state of the biasing member 152, FIG. 20 illustrates the position of the plunger assembly 144 in an intermediate state of the biasing member 152 (shown elsewhere), and FIG. 21 illustrates the position of the plunger assembly 144 in an unloaded state of the biasing member 152. FIGS. 19-21 also illustrate how one of the resistance plungers 150 forces the resistance medium 156 to move in one of the resistance channels 142 as the plunger assembly 144 moves relative to the manifold 122. FIGS. 18-21 further illustrate an exemplary structure of one of the resistance channels 142. More specifically, the resistance channel 142 includes an input channel portion 158 and an output channel portion 160 formed in the upper manifold portion 126. An intermediate channel portion 162 formed in the lower manifold portion 124 couples the input channel portion 158 to the output channel portion 160. As described further below, the resistance plunger 150 forces the resistance medium 156 to move through the input channel portion 158, through the intermediate channel portion 162, and into the output channel portion 160 of the resistance channels 142 as the plunger assembly 144 moves relative to the manifold 122.

One or more characteristics of the biasing member 152, the plunger assembly 144, the manifold 122, the resistance medium 156, and the secondary drug 155 may be selected to provide a desired delivery duration and/or delivery rate of the secondary drug 155 to the patient. These characteristics include, for example, the elastic constant of the biasing member 152, the cross-sectional area and length of the resistance channels 142, the number of resistance plungers 150 and resistance channels 142, the viscosity of the resistance medium 156, the cross-sectional area of the drug reservoir 136, the viscosity of the secondary drug 155, and the cross-sectional area of the manifold drug channel 132. In some embodiments, one or more characteristics may be configured such that the biasing member 152 moves away from the loaded state and toward the unloaded state at a substantially constant rate (for example, constant to within +/-20 percent). For example, the cross-sectional area of the input channel portion 158 may be relatively small compared to the cross-sectional areas of the intermediate channel portion 162 and the output channel portion 160. In these embodiments, the resistance provided by the resistance channels 142 and the resistance medium 156 is largely caused by the input channel portions 158. As the biasing member 152 moves from the loaded state and towards the unloaded state, the resistance plunger 150 moves the resistance medium 156 through the input channel portion 158, the amount of the resistance medium 156 in the input channel portion 158 decreases, and the resistance decreases in a substantially linear manner. Meanwhile, the force exerted by the biasing member 152 decreases in a substantially linear manner. As such, the biasing member 152 may move away from the loaded state and toward the unloaded state at a substantially constant rate, and the secondary drug 155 may be delivered at a substantially constant rate.

In one example, the resistance channels have a round cross-section and contain a viscous, incompressible, Newtonian resistance medium that moves in laminar flow. In this example, movement of the plunger assembly 144, driven by the spring force, is governed by Poiseuille's equation. The flow of drug out of the drug reservoir is given by this equation:

$$Q = (R^2k) / (N8\mu)$$

where Q is the flow rate of the drug out of the drug reservoir, R is the radius of the resistive channel, k is the spring force, N is the number of resistive channels, and $\mu$ is the viscosity of the damping medium. When R, k, N and $\mu$ are constants, the flow rate Q of the drug will likewise be constant.

In another example, a constant delivery rate is facilitated by limiting the unloading of the biasing member 152. For example, the biasing member 152 may have an unloading distance over which the biasing member 152 moves from a fully-loaded state to a fully-unloaded state, but the drug-delivery device assembly 110 may be designed to operate through fifty percent (or some other suitable percentage) of that range, so that the biasing member 152 moves only fifty percent of the unloading distance during use. As such, the force exerted by the biasing member 152 may remain more constant in use, the biasing member 152 may move away from the loaded state and toward the unloaded state at a substantially constant rate, and the secondary drug 155 may be delivered at a substantially constant rate.

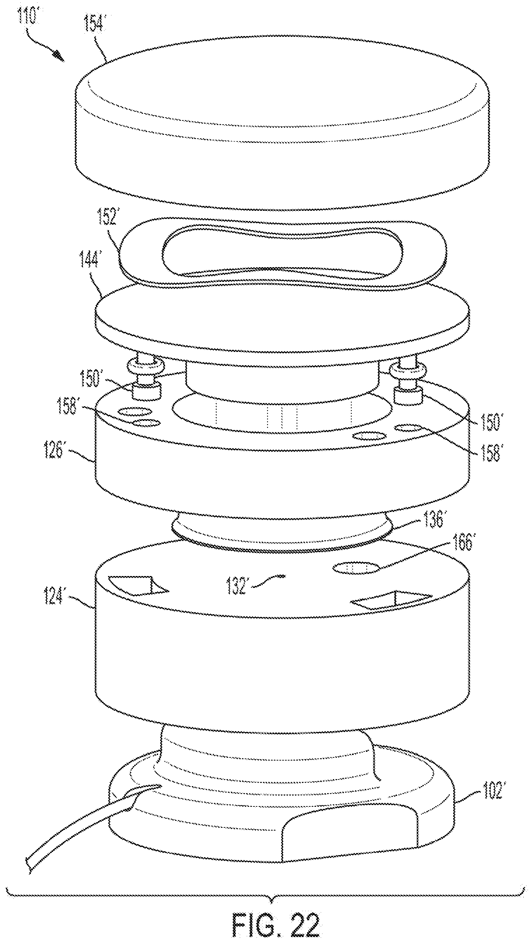

FIGS. 22-26 illustrate another embodiment of a drug-delivery device assembly 110' and an infusion hub 102'. The drug-delivery device assembly 110' and the infusion hub 102' are substantially similar to drug-delivery device assembly 110 and the infusion hub 102, respectively, with like reference numerals indicating like parts, except as described below. Like the infusion hub 102, the infusion hub 102' may receive a primary drug from a primary drug source (shown elsewhere - for example, the primary drug source 106 of FIG. 12). The infusion hub 102' has a recessed upper surface portion 164' to accommodate features of the drug-delivery device assembly 110', as described in further detail below. The following components of the drug-delivery device assembly 110' are shown in FIGS. 22-26: a lower manifold portion 124', an upper manifold portion 126', a plunger assembly 144', a biasing member 152', and a cover 154'.

The drug-delivery device assembly 110' also includes a flexible drug reservoir 136' carried within the upper manifold portion 126', and the flexible drug reservoir 136' carries the secondary drug 155'. The flexible drug reservoir 136' may be made from a thin, flexible, drug-compatible film, such as COP, COC, HDPE, and/or PCTFE. Other materials may also be used, depending upon the secondary drug 155' carried therein. The flexible drug reservoir 136' includes an open proximal side 165' to facilitate (1) initially receiving the secondary drug 155' via a proximally-extending fill port 166' (which is disposed distally relative to the recessed upper surface portion 164' of the infusion hub 102 and receives a stopper 168' after filling the flexible drug reservoir 136'); and (2) delivering the secondary drug 155' to the manifold drug channel 132' of the lower manifold portion 124' and ultimately the patient (when the biasing member 152' moves from a loaded state and toward an unloaded state to move the plunger assembly 144' proximally and compress the flexible drug reservoir 136').

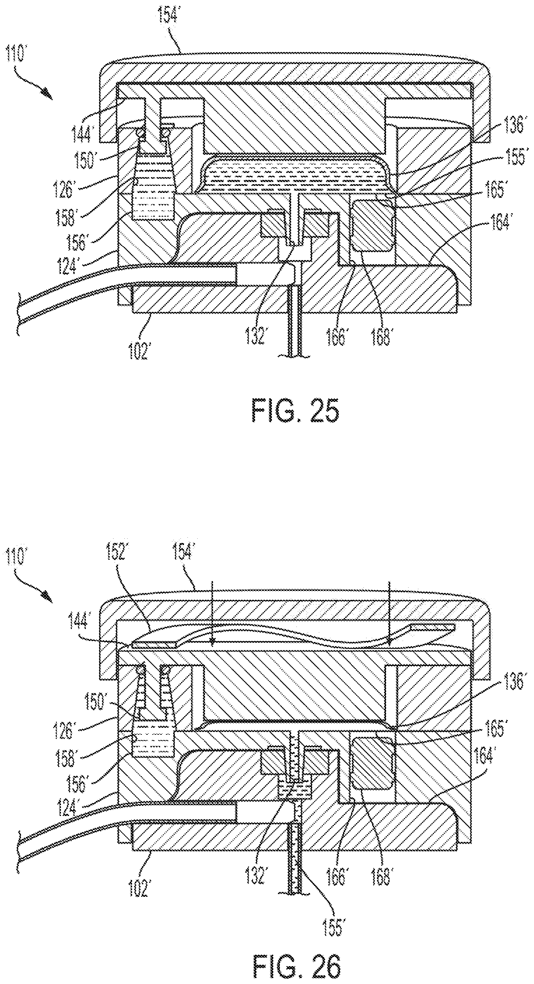

The manifold 122' also includes features that facilitate delivering the secondary drug 155' to a patient at a substantially constant rate. More specifically, each input channel portion 158' of the upper manifold 122' includes a cross-sectional area (for example, a circular area) that increases in the proximal direction. As a resistance plunger 150' moves proximally in such a tapering input channel portion 158', the distance between the resistance plunger 150' and the wall of the input channel portion 158' increases. Tendrils of the resistance medium 156' around the resistance plunger 150' increase in thickness, and the shear stress around the resistance plunger 150' decreases. This decreases the resistance force applied to the plunger assembly 144', which compensates for an increasing shear stress applied to the resistance plunger 150' due to its increasing contact length against the resistance medium 156'. The decreasing resistance force also compensates for a decreasing force exerted by the biasing member 152' as it moves from a loaded state (for example, as shown in FIG. 25) and toward an unloaded state (for example, as shown in FIG. 26; that is, as the biasing member 152' moves the plunger assembly 144' proximally). Overall, these changing forces cause the plunger assembly 144' to move proximally at a substantially constant rate, and the drug-delivery device assembly 110' may thereby deliver the secondary drug 155' to a patient at a substantially constant rate.

The embodiments of the drug-delivery device assemblies 110, 110' described above include drug reservoirs and resistance channels that are arranged in parallel to each other. In some cases, parallel arrangements of drug reservoirs and resistance channels may be advantageous for one or more of the following reasons: (1) such arrangements facilitate providing a low-profile drug-device delivery assembly; (2) such arrangements provide by decoupling the size and location of the resistance channels from those of the drug reservoir; and (3) such arrangements permit the resistance medium to be gradually discharged from the output channel portions. The resulting decrease in shear stress may compensate for the decrease of the force exerted by the biasing member, and a drug-delivery device assembly may thereby deliver the secondary drug to a patient at a substantially constant rate. Alternatively, in some embodiments reservoirs and resistance channels of drug-delivery device assemblies are arranged in series with each other. Exemplary embodiments of such drug-delivery device assemblies are described in further detail below.

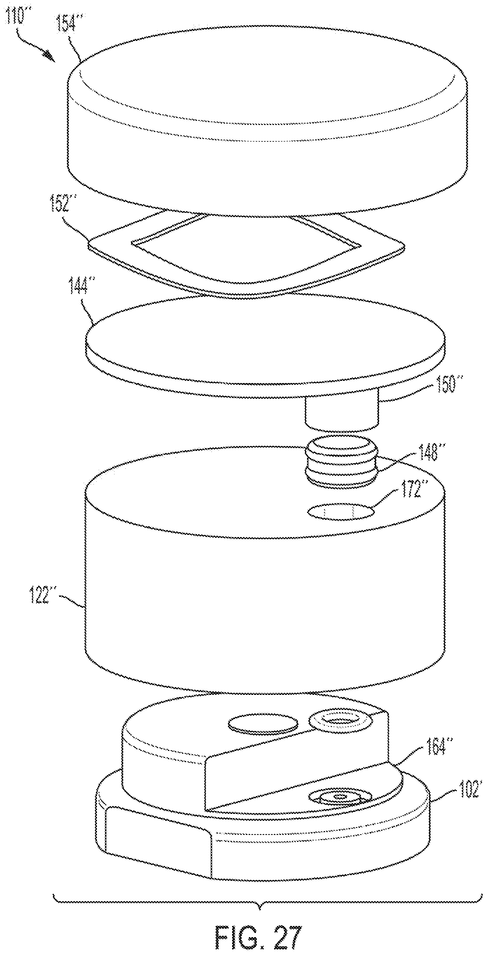

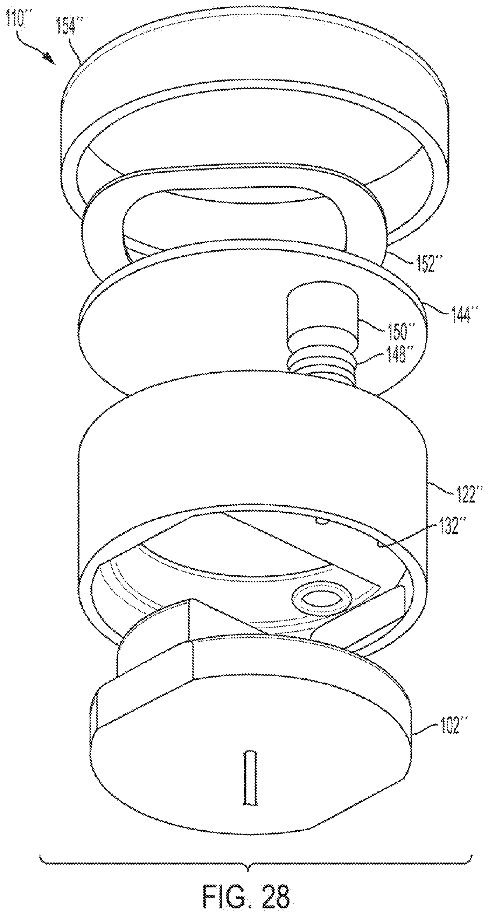

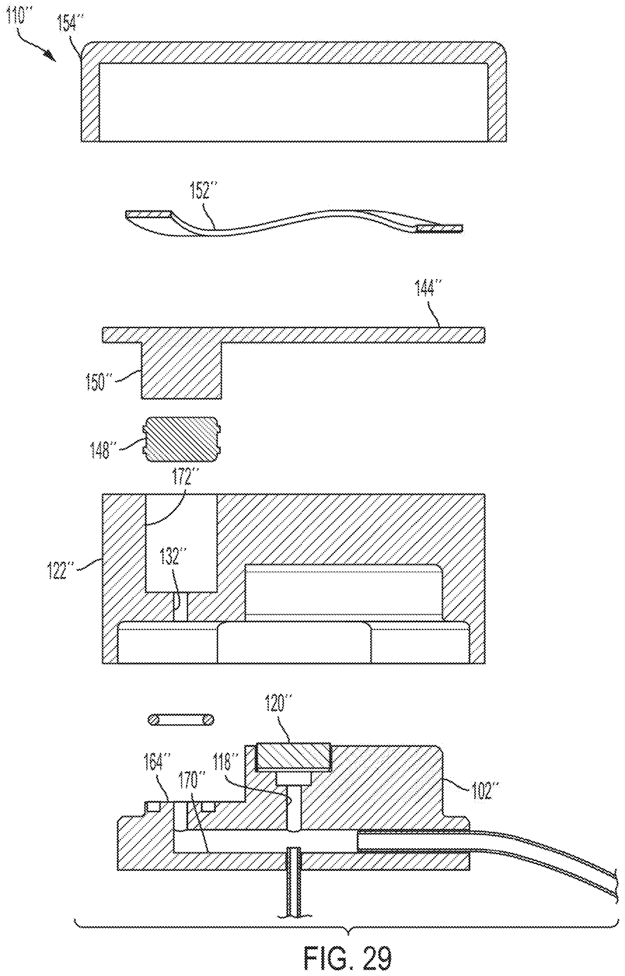

FIGS. 27-31 illustrate another embodiment of a drug-delivery device assembly 110" and an infusion hub 102". The drug-delivery device assembly 110" and the infusion hub 102" are substantially similar to drug-delivery device assembly 110 and the infusion hub 102, respectively, with like reference numerals indicating like parts, except as described below. Like the infusion hub 102, the infusion hub 102" may receive a primary drug from a primary drug source (shown elsewhere - for example, the primary drug source 106 of FIG. 12). The infusion hub 102" has a recessed upper surface portion 164" to accommodate features of the drug-delivery device assembly 110". The infusion hub 102" also includes a generally transversely extending secondary drug channel 170", and the secondary drug channel 118" and the septum 120" may be unused or omitted.

The following components of the drug-delivery device assembly 110" are shown in FIGS. 27-31: a manifold 122", a plunger assembly 144", a biasing member 152", and a cover 154". The manifold 122" includes a manifold drug channel 132" coupled to the secondary drug channel 170" of the infusion hub 102". The manifold drug channel 132" is also coupled to a common reservoir 172". The common reservoir 172" defines both a drug reservoir and a resistance channel. That is, the common reservoir 172" initially carries the secondary drug 155" at a proximal end (that is, adjacent to the manifold drug channel 132"). The drug plunger 148", which is separate from the remainder of the plunger assembly 144", is disposed distally from the secondary drug 155" in the reservoir. The resistance medium 156" is disposed distally from the drug plunger 148" in the common reservoir 172". The resistance plunger 150" is disposed distally from the resistance medium 156" and is also received in the common reservoir 172".

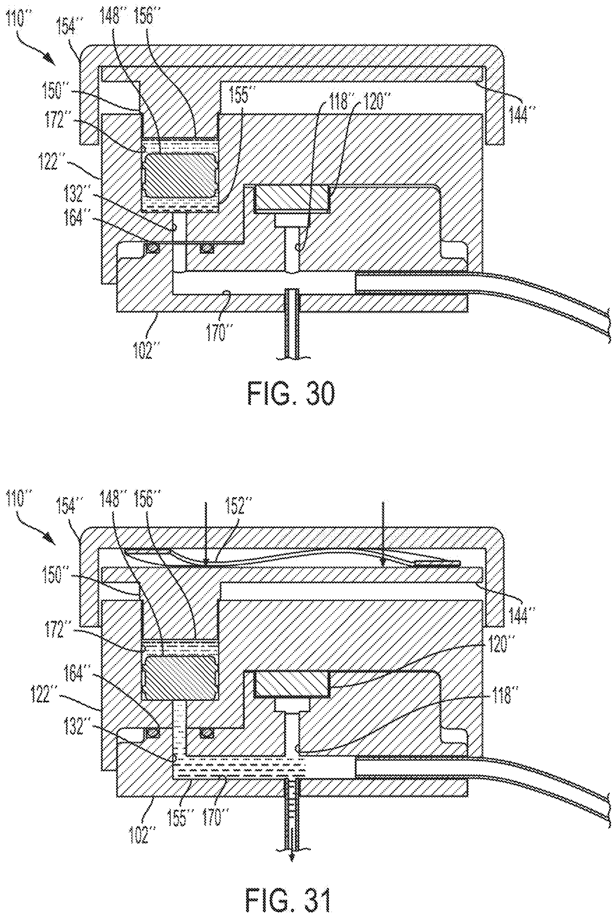

The biasing member 152" may move from a loaded state (for example, as shown in FIG. 30) and toward an unloaded state (for example, as shown in FIG. 31) to move the resistance plunger 150" proximally. The resistance plunger 150" thereby moves the resistance medium 156" proximally while the resistance medium 156" exerts a resistance force in the opposite direction. The resistance medium 156" moves the drug plunger 148" proximally, which forces the secondary drug 155" from the reservoir 172", through the manifold drug channel 132", through the infusion hub 102", and into the patient's body together with any of the primary drug that is also present in the infusion hub 102".

In some embodiments, the infusion hub 102" and the drug-delivery device assembly 110" may advantageously provide a low-profile and simple structure for delivering a relatively low volume dose of the secondary drug 155". In these embodiments, the biasing member 152" may move over a relatively small portion of its unloading distance to deliver the dose of the secondary drug 155". The force exerted by the biasing member 152" may remain substantially constant over such a distance, and the secondary drug 155" is thereby delivered at a substantially constant rate.

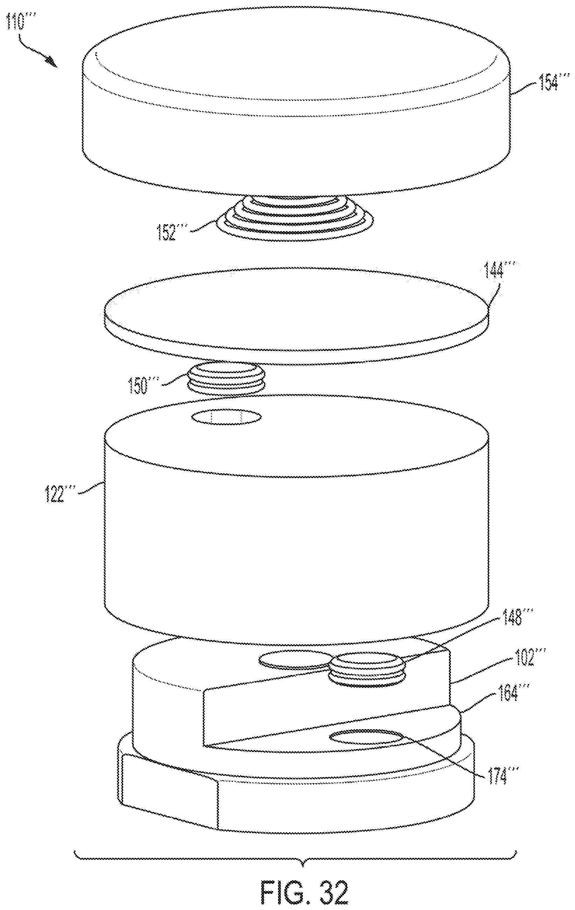

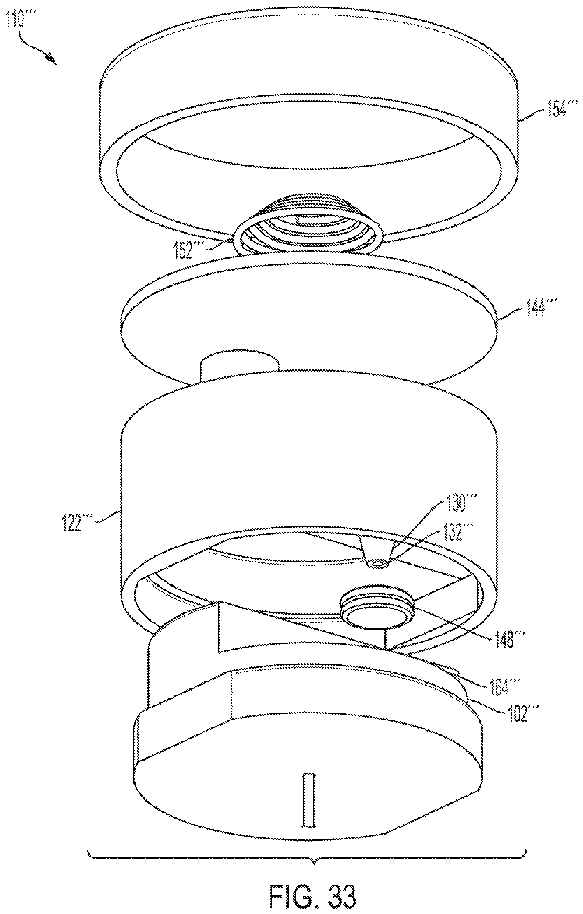

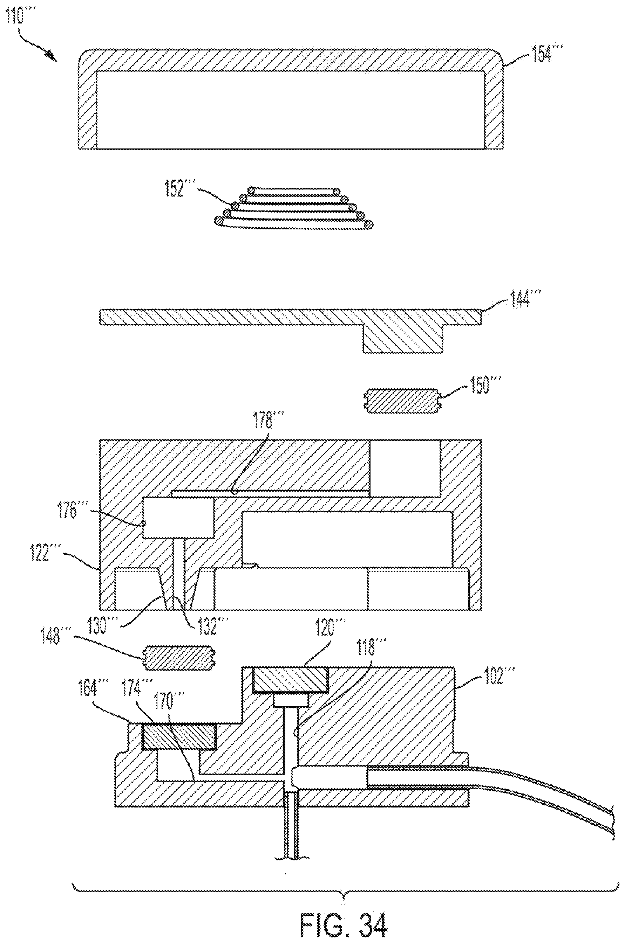

FIGS. 32-36 illustrate another embodiment of a drug-delivery device assembly 110‴ and an infusion hub 102‴. The drug-delivery device assembly 110‴ and the infusion hub 102‴ are substantially similar to drug-delivery device assembly 110 and the infusion hub 102, respectively, with like reference numerals indicating like parts, except as described below. Like the infusion hub 102, the infusion hub 102‴ may receive a primary drug from a primary drug source (shown elsewhere - for example, the primary drug source 106 of FIG. 12). The infusion hub 102‴ has a recessed upper surface portion 164‴ to accommodate features of the drug-delivery device assembly 110‴. The infusion hub 102‴ also includes a generally transversely extending secondary drug channel 170‴ and a septum 174‴, and the secondary drug channel 118‴ and the septum 120‴ may be unused or omitted.

The following components of the drug-delivery device assembly 110‴ are shown in FIGS. 32-36: a manifold 122‴, a plunger assembly 144‴, a biasing member 152‴, and a cover 154‴. The manifold 122‴ includes a protrusion 130‴ that pierces the septum 174‴ of the infusion hub 102‴. The protrusion 130‴ includes a manifold drug channel 132‴ for delivering the secondary drug 155‴ to the secondary drug channel 170‴ of the infusion hub 102‴. The manifold drug channel 132‴ is also coupled to a drug reservoir 176‴. The drug reservoir 176‴ initially carries a secondary drug 155‴ at a proximal end (that is, adjacent to the manifold drug channel 132‴). A drug plunger 148‴, which is separate from the remainder of the plunger assembly 144‴, is disposed distally from the secondary drug 155‴ in the drug reservoir 176‴. The drug reservoir 176‴ is coupled to a transversely extending resistance channel 178‴, and a resistance medium 156‴ is carried in the resistance channel 178‴. The resistance channel 178‴ may have a relatively small cross-sectional area (for example, a circular area) to slow movement of the resistance medium 156‴, which facilitates delivering the secondary drug 155‴ over a relatively long time period. The resistance channel 178‴ also carries a resistance plunger 150‴ distally from the resistance medium 156‴.

The biasing member 152‴ may be, for example and as illustrated in FIGS. 32-36, a compression spring, more specifically a conical spring. In some embodiments, the coils of the conical spring may nest inside one another in the loaded state, which advantageously facilitates providing the drug-delivery device assembly 110‴ with a low-profile structure. Further, the conical spring may move over a relatively small portion of its unloading distance to deliver the dose of the secondary drug 155‴. The force exerted by the biasing member 152‴ may remain substantially constant over such a distance, and the secondary drug 155‴ is thereby delivered at a substantially constant rate.

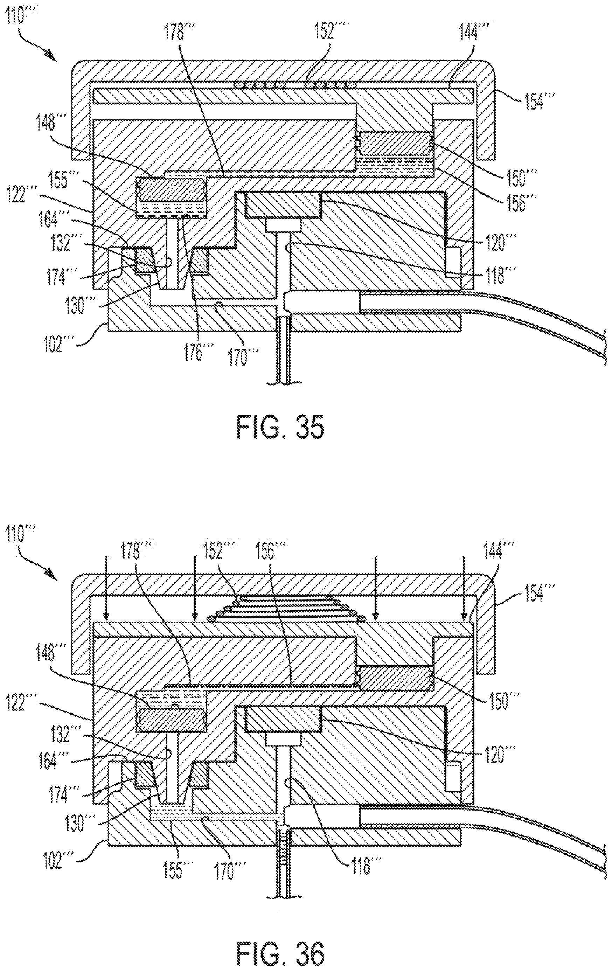

The biasing member 152‴ may move from a loaded state (for example, as shown in FIG. 35) and toward an unloaded state (for example, as shown in FIG. 36) to move the resistance plunger 150‴ proximally. The resistance plunger 150‴ thereby moves the resistance medium 156‴ transversely in the resistance channel 178‴ while the resistance medium 156‴ exerts a resistance force in the opposite direction. The resistance medium 156‴ moves the drug plunger 148‴ proximally, which forces the secondary drug 155‴ from the drug reservoir 176‴, through the manifold drug channel 132‴, through the infusion hub 102‴, and into the patient's body together with any of the primary drug that is also present in the infusion hub 102‴.

In some embodiments, drug-delivery device assemblies according to the present disclosure may be used by patients that are not on a course of infusion treatment (that is, without an infusion hub). In these embodiments, drug-delivery device assemblies may be used with an adapter or provided with a needle or cannula to be inserted directly into the subcutaneous space of the patient.

**Claims**

1. A drug-delivery device assembly (110, 110′, 110″, 110‴) comprising:

   a manifold (122, 122′, 122″, 122‴) comprising a drug channel (132, 132′, 132″, 132‴), the manifold (122, 122′, 122″, 122‴) configured to detachably couple with an infusion hub (102, 102′, 102″, 102‴) configured to subcutaneously deliver a primary drug into a patient's body;
   a drug reservoir (136, 136′) configured to store a secondary drug (155, 155′, 155″, 155‴), the drug reservoir (136, 136′) disposed within the manifold (122, 122′, 122″, 122‴) and coupled to the drug channel (132, 132′, 132″, 132‴);
   a drug plunger (148, 148″, 148‴) being movable relative to the drug reservoir (136, 136′); and
   a biasing member (152, 152′, 152″, 152‴) configured to move away from a loaded state and toward an unloaded state to move the drug plunger (148, 148″, 148‴) relative to the drug reservoir (136, 136′) and force the secondary drug (155, 155′, 155″, 155‴) from the drug reservoir (136, 136′), through the drug channel (132, 132′, 132″, 132‴), through the infusion hub (102, 102′, 102″, 102‴), and into the patient's body;
   **characterized in that** the manifold (122, 122′, 122″, 122‴) further comprises a resistance channel (142, 172″, 178‴) configured to carry a resistance medium (156, 156′, 156″, 156‴), wherein a movement of the resistance medium (156, 156′, 156″, 156‴) in the resistance channel (142, 172″, 178‴) provides resistance to a movement of the biasing member (152, 152′, 152″, 152‴) away from the loaded state and toward the unloaded state.

2. The drug-delivery device assembly (110, 110′, 110″, 110‴) of claim 1, wherein the movement of the resistance medium (156, 156′, 156″, 156‴) in the resistance channel (142, 172″, 178‴) causes the biasing member (152, 152′, 152″, 152‴) to move away from the loaded state and toward the unloaded state at a substantially constant rate.

3. The drug-delivery device assembly (110, 110′) of claim 1, wherein the drug channel (132, 132′) and the resistance channel (142) are arranged in parallel relative to each other.

4. The drug-delivery device assembly (110″, 110‴) of claim 1, wherein the drug channel (132″, 132‴) and the resistance channel (172″, 178‴) are arranged in series relative to each other.

5. The drug-delivery device assembly (110, 110′, 110″, 110‴) of claim 1, further comprising a resistance plunger (150, 150′, 150″, 150‴) being movable in the resistance channel (142, 172″, 178‴) to move the resistance medium (156, 156′, 156″, 156‴) in the resistance channel (142, 172″, 178‴).

6. The drug-delivery device assembly (110, 110′) of claim 5, further comprising a plunger assembly (144, 144′) including the drug plunger (148), the resistance plunger (150, 150'), and a plunger base (146, 146'), the plunger base (146, 146') being coupled to the drug plunger (148) and the resistance plunger (150, 150') such that the drug plunger (148), the resistance plunger (150, 150'), and the plunger base (146, 146') are movable together.

7. The drug-delivery device assembly (110, 110') of claim 6, further comprising a cover (154, 154') coupled to the manifold (122, 122'), wherein the biasing member (152, 152') is disposed between the cover (154, 154') and the plunger base (146, 146').

8. The drug-delivery device assembly (110", 110‴) of claim 5, further comprising a plunger assembly (144", 144‴) including the drug plunger (148", 148‴), the resistance plunger (150", 150‴), and a plunger base, the plunger base being disposed apart from the drug plunger (148", 148‴).

9. The drug-delivery device assembly (110", 110‴) of claim 8, wherein the resistance channel (172", 178‴) is configured to carry the resistance medium (156", 156‴) between the resistance plunger (150", 150‴) and the drug plunger (148", 148‴).

10. The drug-delivery device assembly (110) of claim 1, wherein the drug reservoir (136) is defined by one or more walls of the manifold (122).

11. The drug-delivery device assembly (110') of claim 1, wherein the drug reservoir (136') comprises a flexible drug reservoir carried by the manifold (122').

12. The drug-delivery device assembly (110, 110', 110", 110‴) of claim 1, wherein the biasing member (152, 152', 152", 152‴) comprises an unloading distance over which the biasing member (152, 152', 152", 152‴) moves from a fully-loaded state to a fully-unloaded state, and the drug-delivery device assembly inhibits the biasing member (152, 152', 152", 152‴) from moving more than fifty percent of the unloading distance when causing the drug plunger (148, 148", 148‴) to move relative to the drug reservoir (136, 136') and expel the secondary drug (155, 155', 155", 155‴) from the drug reservoir (136, 136').

13. The drug-delivery device assembly (110, 110', 110", 110‴) of claim 1, wherein the biasing member (152, 152', 152", 152‴) comprises at least one of a wave washer and a conical spring.

14. The drug-delivery device assembly (110, 110', 110", 110‴) of claim 1, wherein the secondary drug (155, 155', 155", 155‴) includes one of an insulin, a glucagon, and a nonsteroidal anti-inflammatory drug (NSAID).

15. A drug-delivery device system comprising:

an infusion hub (102, 102', 102", 102‴) configured to receive a primary drug from a primary drug-delivery device and deliver the primary drug into a patient's body; and
the drug-delivery device assembly (110, 110', 110", 110‴) of claim 1, the drug-delivery device assembly (110, 110', 110", 110‴) being configured to detachably couple with the infusion hub (102, 102', 102", 102‴).

**Patentansprüche**

1. Arzneimittelabgabevorrichtungsanordnung (110, 110', 110", 110‴), umfassend:

einen Verteiler (122, 122', 122", 122‴), umfassend einen Arzneimittelkanal (132, 132', 132", 132‴), wobei der Verteiler (122, 122', 122", 122‴) konfiguriert ist, um mit einem Infusionsknoten (102, 102', 102", 102‴) lösbar gekoppelt zu werden, der konfiguriert ist, um ein Primärarzneimittel in den Körper eines Patienten subkutan abzugeben;
ein Arzneimittelreservoir (136, 136'), das konfiguriert ist, um ein Sekundärarzneimittel (155, 155', 155", 155‴) zu lagern, wobei das Arzneimittelreservoir (136, 136') innerhalb des Verteilers (122, 122', 122", 122‴) angeordnet und mit dem Arzneimittelkanal (132, 132', 132", 132‴) gekoppelt ist;
einen Arzneimittelkolben (148, 148", 148‴), der relativ zu dem Arzneimittelreservoir (136, 136') bewegbar ist; und
ein Vorspannelement (152, 152', 152", 152‴), das konfiguriert ist, um sich von einem belasteten Zustand weg und in einen entlasteten Zustand hinein zu bewegen, um den Arzneimittelkolben (148, 148", 148‴) relativ zu dem Arzneimittelreservoir (136, 136') zu bewegen und das Sekundärarzneimittel (155, 155', 155", 155‴) aus dem Arzneimittelreservoir (136, 136') durch den Arzneimittelkanal (132, 132', 132", 132''') hindurch, durch den Infusionsknoten (102, 102', 102", 102''') hindurch in den Körper des Patienten zu drücken; **dadurch gekennzeichnet, dass** der Verteiler (122, 122', 122", 122''') ferner einen Widerstandskanal (142, 172", 178''') umfasst, der konfiguriert ist, um ein Widerstandsmedium (156, 156', 156", 156''') zu transportieren, wobei eine Bewegung des Widerstandsmediums (156, 156', 156", 156''') in dem Widerstandskanal (142, 172", 178''') einer Bewegung des Vorspannelements (152, 152', 152", 152''') von dem belasteten Zustand weg und in den entlasteten Zustand hinein bereitstellt.

2. Arzneimittelabgabevorrichtungsanordnung (110, 110', 110", 110''') nach Anspruch 1, wobei die Bewegung des Widerstandsmediums (156, 156', 156", 156''') in dem Widerstandskanal (142, 172", 178''') das Vorspannelement (152, 152', 152", 152''') veranlasst, mit einer im Wesentlichen konstanten Geschwindigkeit von dem belasteten Zustand weg und in den entlasteten Zustand hinein zu bewegen.

3. Arzneimittelabgabevorrichtungsanordnung (110, 110') nach Anspruch 1, wobei der Arzneimittelkanal (132, 132') und der Widerstandskanal (142) parallel relativ zueinander angeordnet sind.

4. Arzneimittelabgabevorrichtungsanordnung (110", 110''') nach Anspruch 1, wobei der Arzneimittelkanal (132", 132''') und der Widerstandskanal (172", 178''') in Reihe relativ zueinander angeordnet sind.

5. Arzneimittelabgabevorrichtungsanordnung (110, 110', 110", 110''') nach Anspruch 1, ferner umfassend einen Widerstandskolben (150, 150', 150", 150''') der in dem Widerstandskanal (142, 172", 178''') bewegbar ist, um das Widerstandsmedium (156, 156', 156", 156''') in dem Widerstandskanal (142, 172", 178''') zu bewegen.

6. Arzneimittelabgabevorrichtungsanordnung (110, 110') nach Anspruch 5, ferner umfassend eine Kolbenanordnung (144, 144'), die den Arzneimittelkolben (148), den Widerstandskolben (150, 150') und eine Kolbenbasis (146, 146') einschließt, wobei die Kolbenbasis (146, 146') mit dem Arzneimittelkolben (148) und dem Widerstandskolben (150, 150') derart gekoppelt ist, dass der Arzneimittelkolben (148), der Widerstandskolben (150, 150') und die Kolbenbasis (146, 146') gemeinsam bewegbar sind.

7. Arzneimittelabgabevorrichtungsanordnung (110, 110') nach Anspruch 6, ferner umfassend eine Abdeckung (154, 154'), die mit dem Verteiler (122, 122') gekoppelt ist, wobei das Vorspannelement (152, 152') zwischen der Abdeckung (154, 154') und der Kolbenbasis (146, 146') angeordnet ist.

8. Arzneimittelabgabevorrichtungsanordnung (110", 110''') nach Anspruch 5, ferner umfassend eine Kolbenanordnung (144", 144''') die den Arzneimittelkolben (148", 148'''), den Widerstandskolben (150", 150''') und eine Kolbenbasis einschließt, wobei die Kolbenbasis von dem Arzneimittelkolben (148", 148''') getrennt angeordnet ist.

9. Arzneimittelabgabevorrichtungsanordnung (110", 110''') nach Anspruch 8, wobei der Widerstandskanal (172", 178''') konfiguriert ist, um das Widerstandsmedium (156", 156''') zwischen dem Widerstandskolben (150", 150''') und dem Arzneimittelkolben (148", 148''') zu transportieren.

10. Arzneimittelabgabevorrichtungsanordnung (110) nach Anspruch 1, wobei das Arzneimittelreservoir (136) durch eine oder mehrere Wände des Verteilers (122) definiert ist.

11. Arzneimittelabgabevorrichtungsanordnung (110') nach Anspruch 1, wobei das Arzneimittelreservoir (136') ein flexibles Arzneimittelreservoir umfasst, das durch den Verteiler (122') getragen wird.

12. Arzneimittelabgabevorrichtungsanordnung (110, 110', 110", 110''') nach Anspruch 1, wobei das Vorspannelement (152, 152', 152", 152''') eine Entlastungsdistanz umfasst, über die sich das Vorspannelement (152, 152', 152", 152''') von einem vollständig belasteten Zustand in einen vollständig entlasteten Zustand bewegt, und die Arzneimittelabgabevorrichtungsanordnung das Vorspannelement (152, 152', 152", 152''') daran hindert, sich mehr als fünfzig Prozent der Entlastungsdistanz zu bewegen, wenn der Arzneimittelkolben (148, 148", 148''') veranlasst wird, sich relativ zu dem Arzneimittelreservoir (136, 136') zu bewegen und das Sekundärarzneimittel (155, 155', 155", 155''') aus dem Arzneimittelreservoir (136, 136') auszustoßen.

13. Arzneimittelabgabevorrichtungsanordnung (110, 110', 110", 110''') nach Anspruch 1, wobei das Vorspannelement (152, 152', 152", 152''') mindestens eine Wellscheibe und eine konische Feder umfasst.

**14.** Arzneimittelabgabevorrichtungsanordnung (110, 110', 110", 110‴) nach Anspruch 1, wobei das Sekundärarzneimittel (155, 155', 155", 155‴) eines von Insulin, Glucagon oder einem nichtsteroidalen Antirheumatikum (NSAID) einschließt.

**15.** Arzneimittelabgabesystem, umfassend:

einen Infusionsknoten (102, 102', 102", 102‴), der konfiguriert ist, um ein Primärarzneimittel von einer Primärarzneimittelabgabevorrichtung aufzunehmen und das Primärarzneimittel in den Körper eines Patienten abzugeben; und

die Arzneimittelabgabevorrichtungsanordnung (110, 110', 110", 110‴) nach Anspruch 1, wobei die Arzneimittelabgabevorrichtungsanordnung (110, 110', 110", 110‴) konfiguriert ist, um mit dem Infusionsknoten (102, 102', 102", 102‴) lösbar gekoppelt zu werden.

## Revendications

**1.** Ensemble de dispositifs d'administration de médicaments (110, 110', 110", 110‴) comprenant :

un collecteur (122, 122', 122", 122‴) comprenant un canal de médicament (132, 132', 132", 132‴), le collecteur (122, 122', 122", 122‴) étant conçu pour se coupler de manière amovible à un concentrateur de perfusion (102, 102', 102", 102‴) conçu pour administrer par voie sous-cutanée un médicament primaire dans le corps d'un patient ;

un réservoir de médicament (136, 136') conçu pour stocker un médicament secondaire (155, 155', 155", 155‴), le réservoir de médicament (136, 136') étant disposé à l'intérieur du collecteur (122, 122', 122", 122‴) et couplé au canal de médicament (132, 132', 132", 132‴) ;

un piston de médicament (148, 148", 148‴) mobile par rapport au réservoir de médicament (136, 136') ; et

un élément de sollicitation (152, 152', 152", 152‴) conçu pour s'éloigner d'un état chargé et se rapprocher d'un état non chargé afin de déplacer le piston de médicament (148, 148", 148‴) par rapport au réservoir de médicament (136, 136') et forcer le médicament secondaire (155, 155', 155", 155‴) à sortir du réservoir de médicament (136, 136'), à travers le canal de médicament (132, 132', 132", 132‴), à travers le concentrateur de perfusion (102, 102', 102", 102‴), et à pénétrer dans le corps du patient ;

**caractérisé en ce que** le collecteur (122, 122', 122", 122‴) comprend en outre un canal de résistance (142, 172", 178‴) conçu pour transporter un agent de résistance (156, 156', 156", 156‴), dans lequel un mouvement de l'agent de résistance (156, 156', 156", 156‴) dans le canal de résistance (142, 172", 178‴) fournit une résistance à un mouvement de l'élément de sollicitation (152, 152', 152", 152‴) à partir de l'état chargé et vers l'état déchargé.

**2.** Ensemble de dispositifs d'administration de médicaments (110, 110', 110", 110‴) selon la revendication 1, dans lequel le mouvement du milieu de résistance (156, 156', 156", 156‴) dans le canal de résistance (142, 172", 178‴) fait que l'élément de sollicitation (152, 152', 152", 152‴) s'éloigne de l'état chargé et se rapproche de l'état déchargé à une vitesse sensiblement constante.

**3.** Ensemble de dispositifs d'administration de médicaments (110, 110') selon la revendication 1, dans lequel le canal de médicament (132, 132') et le canal de résistance (142) sont disposés parallèlement l'un par rapport à l'autre.

**4.** Ensemble de dispositifs d'administration de médicaments (110", 110‴) selon la revendication 1, dans lequel le canal de médicament (132", 132‴) et le canal de résistance (172", 178‴) sont disposés en série l'un par rapport à l'autre.

**5.** Ensemble de dispositifs d'administration de médicaments (110, 110', 110", 110‴) selon la revendication 1, comprenant en outre un piston de résistance (150, 150', 150", 150‴) mobile dans le canal de résistance (142, 172", 178‴) pour déplacer le milieu de résistance (156, 156', 156", 156‴) dans le canal de résistance (142, 172", 178‴).

**6.** Ensemble de dispositifs d'administration de médicaments (110, 110') selon la revendication 5, comprenant en outre un ensemble de piston (144, 144') comportant le piston de médicament (148), le piston de résistance (150, 150') et une base de piston (146, 146'), la base de piston (146, 146') étant couplée au piston de médicament (148) et au piston de résistance (150, 150') de sorte que le piston de médicament (148), le piston de résistance (150, 150') et la base du piston (146, 146') sont mobiles ensemble.

7.  Ensemble de dispositifs d'administration de médicaments (110, 110') selon la revendication 6, comprenant en outre un couvercle (154, 154') couplé au collecteur (122, 122'), dans lequel l'élément de sollicitation (152, 152') est disposé entre le couvercle (154, 154') et la base du piston (146, 146').

8.  Ensemble de dispositifs d'administration de médicaments (110", 110"') selon la revendication 5, comprenant en outre un ensemble de piston (144", 144"') comportant le piston de médicament (148", 148"'), le piston de résistance (150", 150"'), et une base de piston, la base de piston étant disposée à l'écart du piston de médicament (148", 148"').

9.  Ensemble de dispositifs d'administration de médicaments (110", 110‴) selon la revendication 8, dans lequel le canal de résistance (172", 178"') est conçu pour transporter le milieu de résistance (156", 156"') entre le piston de résistance (150", 150"') et le piston de médicament (148", 148"').

10. Ensemble de dispositifs d'administration de médicaments (110) selon la revendication 1, dans lequel le réservoir de médicaments (136) est défini par une ou plusieurs parois du collecteur (122).

11. Ensemble de dispositifs d'administration de médicaments (110') selon la revendication 1, dans lequel le réservoir de médicaments (136') comprend un réservoir de médicaments flexible porté par le collecteur (122').

12. Ensemble de dispositifs d'administration de médicaments (110, 110', 110", 110‴) selon la revendication 1, dans lequel l'élément de sollicitation (152, 152', 152", 152"') comprend une distance de déchargement sur laquelle l'élément de sollicitation (152, 152', 152", 152"') se déplace d'un état complètement chargé à un état complètement déchargé, et l'ensemble de dispositifs d'administration de médicaments empêche l'élément de sollicitation (152, 152", 152", 152"') de se déplacer à plus de cinquante pour cent de la distance de déchargement lorsqu'il provoque le déplacement du piston à médicament (148, 148", 148"') par rapport au réservoir à médicament (136, 136') et l'expulsion du médicament secondaire (155, 155', 155", 155"') du réservoir à médicament (136, 136').

13. Ensemble de dispositifs d'administration de médicaments (110, 110', 110", 110‴) selon la revendication 1, dans lequel l'élément de sollicitation (152, 152', 152", 152"') comprend au moins une rondelle ondulée et un ressort conique.

14. Ensemble de dispositifs d'administration de médicaments (110, 110', 110", 110‴) selon la revendication 1, dans lequel le médicament secondaire (155, 155', 155", 155"') comporte une insuline, un glucagon et un anti-inflammatoire non stéroïdien (NSAID).

15. Système de dispositifs d'administration de médicaments comprenant :

    un concentrateur de perfusion (102, 102', 102", 102‴) conçu pour recevoir un médicament primaire d'un dispositif d'administration de médicament primaire et administrer le médicament primaire dans le corps d'un patient ; et l'ensemble de dispositifs d'administration de médicaments (110, 110', 110", 110"') selon la revendication 1, l'ensemble de dispositifs d'administration de médicaments (110, 110', 110", 110"') étant conçu pour s'accoupler de manière amovible avec le concentrateur de perfusion (102, 102', 102", 102"').

FIG. 1

FIG. 2

FIG. 3

FIG. 4

FIG. 5

FIG. 6

FIG. 7A (1) ATTACH INFUSION SET

FIG. 7B (2) GET DRUG-DELIVERY DEVICE ASSEMBLY FROM PACKAGE

FIG. 7C (3) PUSH NEEDLE HUB IN

FIG. 7D (4) REMOVE NEEDLE SHIELD

FIG. 7E (5) ATTACH ASSEMBLY TO INFUSION SET HUB

FIG. 7F (6) TWIST SCREW CAP TO ADMINISTER DOSE

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

FIG. 31

FIG. 32

FIG. 33

FIG. 34

FIG. 35

FIG. 36

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012203198 A1
- WO 2012072555 A1